# EUROPEAN PATENT SPECIFICATION

(11) **EP 4 476 525 B1**
(45) Date of publication and mention of the grant of the patent: **22.10.2025**
(21) Application number: 23703456.6
(22) Date of filing: 09.02.2023
(51) Int. Cl.: G01N 21/27, G01N 21/78, G01N 21/84, G16H 10/40, G16H 30/40, G16H 50/20, G16H 50/70, G01N 21/75, G01N 21/77

(54) **METHOD OF DETERMINING A COLOR EXPECTATION RANGE FOR ASSESSING THE PLAUSIBILITY OF AN ASSUMED REACTION TIME VALUE USED IN AN ANALYTICAL MEASUREMENT BASED ON A COLOR FORMATION REACTION, SYSTEM AND MOBILE DEVICE**
VERFAHREN ZUR BESTIMMUNG EINES FARBERWARTUNGSBEREICHES ZUR BEWERTUNG DER PLAUSIBILITÄT EINES ANGENOMMENEN REAKTIONSZEITWERTES, DER BEI EINER ANALYTISCHEN MESSUNG AUF DER BASIS EINER FARBBILDUNGSREAKTION VERWENDET WIRD, SYSTEM UND MOBILES GERÄT
MÉTHODE DE DÉTERMINATION D'UN INTERVALLE D'ATTENTE DE COULEUR POUR ÉVALUER LA PLAUSIBILITÉ D'UNE VALEUR SUPPOSÉE DE TEMPS DE RÉACTION UTILISÉE DANS UNE MESURE ANALYTIQUE BASÉE SUR UNE RÉACTION DE FORMATION DE COULEUR, SYSTÈME ET DISPOSITIF MOBILE

(30) Priority: 11.02.2022 EP 22156296
(43) Date of publication of application: 18.12.2024
(73) Proprietor: F. Hoffmann-La Roche AG, 4070 Basel (CH); Roche Diabetes Care GmbH, 68305 Mannheim (DE)
(72) Inventor: BERG, Max, 68305 Mannheim (DE); HAILER, Fredrik, 68305 Mannheim (DE); LIMBURG, Bernhard, 68305 Mannheim (DE)
(74) Representative: Riwotzki, Karsten
(86) International application number: PCT/EP2023/053145
(87) International publication number: WO 2023/152200

(56) References cited:
- EP-A1- 3 667 301
- US-A1- 2006 246 574
- US-A1- 2014 065 647
- US-A1- 2017 098 137
- US-A1- 2021 299 651

## Description

### Technical Field

The present invention refers to a determination method of determining at least one color expectation range for assessing the plausibility of an assumed reaction time value. The invention further relates to a measurement method of performing an analytical measurement based on a color formation reaction. Further, the invention relates to a determination system and to a mobile device as well as to computer programs, computer-readable storage media and a kit. The methods, systems, mobile devices, the computer programs, the computer-readable storage media and the kit may be used specifically in medical diagnostics, in order to for example quantitatively or qualitatively detect one or more analytes in one or more body fluids and/or bodily fluids, such as for detecting glucose in blood and/or interstitial fluid. Other fields of application of the present invention, however, are feasible.

### Background art

**In** the field of medical diagnostics, in many cases, one or more analytes have to be detected in samples of a body fluid, such as blood, interstitial fluid, urine, saliva or other types of body fluids. Examples of analytes to be detected are glucose, triglycerides, lactate, cholesterol or other types of analytes typically present in these body fluids. According to the con-centration and/or the presence of the analyte, an appropriate treatment may be chosen, if necessary. Without narrowing the scope, the invention specifically may be described with respect to blood glucose measurements. It shall be noted, however, that the present invention may also be used for other types of analytical measurements using test elements.

Generally, devices and methods known to the skilled person make use of test elements and/or test strips comprising one or more test chemicals, which, in presence of the analyte to be detected, are capable of performing one or more detectable detection reactions, such as optically detectable detection reactions. With regard to the test chemicals comprised in test elements and/or test strips, reference may be made e.g. to J. Hoenes et al.: The Technology Behind Glucose Meters: Test Strips, Diabetes Technology & Therapeutics, Volume 10, Supplement 1, 2008, S-10 to S-26. Other types of test chemistry are possible and may be used for performing the present invention.

In analytical measurements, specifically analytical measurements based on color formation reactions, one technical challenge resides in the evaluation of the color change which is due to the detection reaction. Besides using dedicated analytical devices, such as handheld blood glucose meters, the use of generally available electronics such as smart phones and portable computers or other mobile devices has become more and more popular over the recent years. Thus, a camera comprised by these mobile devices may be used to measure the color change of the detection reaction in the test elements and/or test strips. As opposed to laboratory measurements and measurements performed by using dedicated pairings of analytical measurement devices and test elements and/or test strips, when using mobile computing devices such as smart phones, various influences need to be taken into account. As an example, lighting conditions, positioning, vibrations, electronic defects or other more or less uncontrollable influences are to be considered. Generally, procedures may be used which comprise mathematically correcting and/or compensating consequences of such influences on the color change by making use of color reference for photometric measurements.

US 2020/0249220 A1 describes a method of detecting a urine test strip from a photographed image of the urine test strip. The disclosed method of detecting a urine test strip includes: receiving input of a urine test strip image, which is a photographed image of a urine test strip including a first and a second marker; detecting a first and a second marker image within the urine test strip image; detecting an area between the first and second marker images within the urine test strip image; and detecting a reagent pad and a colorimetric table in the urine test strip image by matching an area of interest, which represents a position of the reagent pad and the colorimetric table in the urine test strip image, with the area between the first and second marker images.

US 9,787,815 B2 describes a method for obtaining a point-of-collection, selected quantitative indicia of an analyte on a test strip using a smartphone that involves imaging a test strip on which a colorimetric reaction of a target sample has occurred due to test strip illumination by the smartphone. The smartphone includes a smartphone app and a smartphone accessory that provides an external environment-independent/internal light-free, imaging environment independent of the smartphone platform being used. The result can then be presented quantitatively or turned into a more consumer-friendly measurement (positive, negative, above average, etc.), displayed to the user, stored for later use, and communicated to a location where practitioners can provide additional review. Additionally, social media integration can allow for device results to be broadcast to specific audiences, to compare healthy living with others, to compete in health based games, create mappings, and other applications.

EP 2 916 117 A1 describes color quantification of chemical test pads and titration of analytes that can be performed under different lighting conditions. In one embodiment, the lighting condition is estimated under which a digital image is captured and utilized to select a set of reference colors from which the quantified color is compared to determine the titration. In another embodiment, a plurality of comparisons is made with different lighting conditions with the result having the highest confidence level being selected to determine the titration.

EP 1 963 828 B1 describes a method for measuring a concentration of an analyte contained in a sample of a biological fluid. In said method, a test strip is provided which comprises at least one test point and at least one reference color section encompassing the color white and/or a color scale. The fluid sample is brought in contact with the test point, and a color indicator is disposed on the test point in accordance with the concentration of the analyte. A camera is placed on the test strip. At least one measured value is detected for the relative position between the camera and the test strip and is compared to a set value range. If the measured value deviates from the set value range, the camera is moved relative to the test strip to reduce the deviation. A colored image on which at least the color indicator and the reference color section are represented is detected with the aid of the camera. The image areas assigned to the color indicator and the color matching section are located, and the color values of said image areas are determined. The analyte concentration in the sample is determined based on the color values with the aid of predefined comparative values.

US 2015/0241358 A1 describes in one embodiment an apparatus for automatic test diagnosis of a test paddle. The apparatus comprises a personal computing device including: a camera to capture images over time of test pads of a test paddle, a processor coupled to the camera, and a display device coupled to the processor. The processor analyzes the color changes over time of each test pad to determine a color trajectory over time for each test pad. The processor compares the color evolution trajectory for each test pad with color calibration curves for each test pad to determine an analyte concentration of a test biological sample, such as urine. During the analysis by the processor, the display device displays a user interface with results of the analyte concentration in response to the analysis over time.

US 2014/065647 A1 describes a system and method for spatiotemporally analyzed rapid assays.

EP 3 667 301 A1 describes a method an system for determining concentration of an analyte in a sample of a bodily fluid, and a method and system for generating a software implemented module.

US 2021/299651 A1 describes a multi-factor urine test system that adjusts for lighting and timing.

US 2017/098137 A1 describes a method, apparatus and system for detecting and determining compromised reagent pads by quantifying color changes induced by exposure to a hostile environment.

US 2006/246574 A1 describes a dispenser for building a lateral flow immunoassay device comprising a fluid supply assembly and a jetting assembly.

Despite the advantages achieved by the known methods and devices, several technical challenges remain. Specifically, user-dependent influences, such as wrongful handling habits and/or incorrect time specification on sample application or other more or less uncontrollable user-dependent handling errors may lead to undetected color changes. Such undetected color changes may result in an inaccurate analytical measurement when determining the analyte concentration based on a color formation reaction.

### Problem to be solved

It is therefore desirable to provide methods and devices that at least partially address the above-mentioned technical challenges. Specifically, it is desirable to provide methods and devices which allow for a detection of color changes introduced by wrongful and/or inappropriate user handling.

### Summary

This problem is addressed by a determination method of determining at least one color expectation range for assessing the plausibility of an assumed reaction time value and by a measurement method of performing an analytical measurement based on a color formation reaction, as defined in appended independent claim 1. Further, by a determination system and a mobile device with the features of the independent claims 13 and 16. Dependent claim 17 is directed to a kit comprising the mobile device according to claim 16.

The scope of the invention is solely defined by the appended claims. Any embodiment in accordance with the invention must fall within the scope of the invention defined by the appended claims.

Advantageous embodiments which might be realized in an isolated fashion or in any arbitrary combinations are listed in the dependent claims as well as throughout the specification.

As used in the following, the terms "have", "comprise" or "include" or any arbitrary grammatical variations thereof are used in a non-exclusive way. Thus, these terms may both refer to a situation in which, besides the feature introduced by these terms, no further features are present in the entity described in this context and to a situation in which one or more further features are present. As an example, the expressions "A has B", "A comprises B" and "A includes B" may both refer to a situation in which, besides B, no other element is present in A (i.e. a situation in which A solely and exclusively consists of B) and to a situation in which, besides B, one or more further elements are present in entity A, such as element C, elements C and D or even further elements.

Further, it shall be noted that the terms "at least one", "one or more" or similar expressions indicating that a feature or element may be present once or more than once typically will be used only once when introducing the respective feature or element. In the following, in most cases, when referring to the respective feature or element, the expressions "at least one" or "one or more" will not be repeated, non-withstanding the fact that the respective feature or element may be present once or more than once.

Further, as used in the following, the terms "preferably", "more preferably", "particularly", "more particularly", "specifically", "more specifically" or similar terms are used in conjunction with optional features, without restricting alternative possibilities. Thus, features introduced by these terms are optional features and are not intended to restrict the scope of the claims in any way. Similarly, features introduced by "in an embodiment of the invention" or similar expressions are intended to be optional features, without any restriction regarding alternative embodiments of the invention, without any restrictions regarding the scope of the invention and without any restriction regarding the possibility of combining the features introduced in such way with other optional or non-optional features of the invention.

In a first aspect of the invention, a determination method of determining at least one color expectation range for assessing the plausibility of an assumed reaction time value used in an analytical measurement based on a color formation reaction is disclosed. The determination method comprises the following steps that, as an example, may be performed in the given order. It shall be noted, however, that a different order may generally also be possible. Further, it may also be possible to perform one or more of the method steps once or repeatedly. Further, it may also be possible to perform two or more of the method steps simultaneously or in a timely overlapping fashion. The determination method may comprise further method steps that are not listed.

The determination method comprises:
a) providing a training set of optical test strips, each optical test strip having a reagent test region;
b) providing a training set of samples of bodily fluids and applying at least one of the samples of bodily fluid to the reagent test region of each optical test strip of the training set of optical test strips;
c) capturing, by at least one mobile device having at least one camera, a training set of images comprising:
   - a first training subset of images, the first training subset of images comprising images of at least one part of at least some of the reagent test regions of the training set of optical test strips having the sample of bodily fluid applied thereto captured at in-time capture time values, the capture time value referring to the time that has passed between the application of the sample in step b) and the capturing of the image;
   - at least one second training subset of images, the at least one second training subset of images comprising images of at least one part of at least some of the reagent test regions of the training set of optical test strips having the sample of bodily fluid applied thereto captured at delayed capture time values;
d) determining, specifically by using at least one processor, more specifically a processor of the mobile device, a training set of color formation values from the images of the training set of images, the training set of color formation values comprising color formation values of at least one color channel for the color formation of the reagent test region of the optical test strips of the training set of optical test strips for in-time capture time values and for delayed capture time values; and
e) deriving the at least one color expectation range for the at least one color channel from the training set of color formation values, the color expectation range defining an expected range of color formation values for in-time capture time values and tolerably delayed capture time values, wherein for the tolerably delayed capture time values the delay *d* does not exceed at least one predefined expiry threshold *dₘₐₓ.*

The term "an analytical measurement based on a color formation reaction" as used herein is a broad term and is to be given its ordinary and customary meaning to a person of ordinary skill in the art and is not to be limited to a special or customized meaning. The term specifically may refer, without limitation, to a quantitative and/or qualitative determination of at least one analyte in an arbitrary sample or aliquot of bodily fluid by using a color formation reaction. For example, the bodily fluid may comprise one or more of blood, interstitial fluid, urine, saliva or other types of body fluids. The result of the determining of the analyte, as an example, may be a concentration of the analyte and/or the presence or absence of the analyte to be determined. Specifically, as an example, the analytical measurement may be a blood glucose measurement, thus the result of the analytical measurement may for example be a blood glucose concentration. In particular, an analytical measurement result value, such as the concentration of the analyte in the bodily fluid, may be determined by the analytical measurement by using a color formation reaction, such as a color-change reaction in response to a quantitative and/or qualitative presence or absence of the analyte in the bodily fluid.

For example, the bodily fluid may comprise one or more of blood, interstitial fluid, urine, saliva or other types of body fluids. Consequently, the term "sample of bodily fluid" may specifically refer to an arbitrary aliquot part or aliquant part of a biological fluid which directly is a bodily fluid or which is derived from a bodily fluid such as by one or more pre-processing steps, e.g. by centrifugation. As an example, the sample of bodily fluid may be a droplet of a body fluid as gathered from the body of a person, such as a droplet of blood and/or interstitial fluid generated, e.g., by piercing of a skin portion of the person, e.g. with a lancet, a needle or the like. The sample of bodily fluid may also be simply referred to as the sample.

In the analytical measurement based on a color formation reaction specifically an optical test strip is used. The term "optical test strip" as used herein is a broad term and is to be given its ordinary and customary meaning to a person of ordinary skill in the art and is not to be limited to a special or customized meaning. The term specifically may refer, without limitation, to an arbitrary element or device configured for performing a color-change detection reaction. The optical test strip may also be referred to as test strip or test element, wherein all three terms may refer to the same element. The optical test strip may particularly have a reagent test region, also referred to as test region and/or test field, containing at least one test chemical for detecting at least one analyte. Specifically, the test chemical may be configured for performing a color-change, e.g. changing its color, due to a presence of the analyte. The color-change may for example depend on a concentration of the analyte in the sample of bodily fluid. **In** particular, the reagent test region may have a visually detectable edge, such as a detectable rim and/or border, contrasting the reagent test region from other parts of the optical test strip. The optical test strip, as an example, may comprise at least one substrate, such as at least one carrier, with the at least one test field applied thereto or integrated therein. **In** particular, the optical test strip may further comprise at least one white area, such as a white field, specifically in a proximity to the reagent test region, for example enclosing or surrounding the test region. **In** particular, a contrast between the reagent test region and the white area surrounding the reagent test region may be sufficient to allow visually detecting an edge and/or rim of the reagent test region. Additionally or alternatively, the substrate or carrier itself may be or may comprise the white area. As an example, the at least one carrier may be strip-shaped, thereby rendering the test element a test strip. These test strips are generally widely in use and available. One test strip may carry a single reagent test region or a plurality of test regions having identical or different test chemicals comprised therein.

The term "an assumed reaction time value used in an analytical measurement based on a color formation reaction" as used herein is a broad term and is to be given its ordinary and customary meaning to a person of ordinary skill in the art and is not to be limited to a special or customized meaning. The term specifically may refer, without limitation, to a numerical indication of a reaction time that is estimated in an analytical measurement. **In** particular, the reaction time value may refer to a time that has passed for a color-change reaction of the reagent test region of the optical test strip. As an example, the assumed reaction time value may, in the analytical measurement, be provided by a user, e.g. a user performing the analytical measurement. **In** particular, in the measurement, the user may be required to provide information, such as a time of sample application onto the reagent test region, allowing to assume and/or predict a reaction time value also referred to as the assumed reaction time value. **In** particular, the assumed reaction time value may refer to an assumed and/or estimated time between sample application to the reagent test region and the measurement, i.e. between a triggering of the color change reaction and a capturing of an image of the reagent test region. Thus, as an example the assumed reaction time value may also be referred to as an assumed capture time value, such as an assumed value for a capture time value, as will be outlined below. The term "assumed reaction time value", or synonymously the term "assumed capture time value", as used herein is a broad term is to be given its ordinary and customary meaning to a person of ordinary skill in the art and is not to be limited to a special or customized meaning. The term specifically may refer, without limitation, to an assumed and/or predicted numerical indication of a reaction time, i.e. of a time during which a chemical reaction, specifically a color-change reaction, has happened. In particular, the assumed reaction time value may refer to a time during which a chemical reaction has happened and may thus also be referred to as chemical reaction timing and/or color-change reaction timing. The assumed reaction time value may specifically be an estimated and/or predicted time value, e.g. based on an indication provided by a user. Thus, the assumed reaction time value may differ from the actual reaction time that may have passed for the color-change reaction of the reagent test region. In the analytical measurement, the assumed reaction time value may be used for determining the analyte concentration from a color formation value. Additionally or alternatively, the assumed reaction time value may refer to an assumed and/or predicted numerical indication of a reaction time range, i.e. of a time range during which the chemical reaction, specifically the color-change reaction, has happened. Thus, as an example, instead of indicating a numerical value in time, such as for example "18 seconds", the assumed reaction time value may additionally or alternatively indicate a numerical range of time, such as a time range, e.g. "within 1 minute" or "between 13 and 45 seconds".

The term "color formation value" may refer to an arbitrary numerical indication, such as numerical representation, of the color of the reagent test region of the test strip, specifically resulting from the color-change detection reaction of the test chemical. Specifically, the color numerically indicated by the color formation value may correlate with an analyte concentration of the sample of bodily fluid applied to the respective optical test strip. Thus, as an example, the color and therefore the color formation value may correlate with a blood glucose value and/or blood glucose concentration. Further, the color of the reagent test region, and thus the color formation value, may change over time. Thus, the correlation between the color formation value and the analyte concentration may further be time-dependent. Therefore, in the analytical measurement, the analyte concentration may be determined from the color formation value by further using the assumed reaction time value.

As an example, the correlation may be represented by one or more functions, wherein the analyte concentration may be determined by inserting into the function the assumed reaction time value and the color formation value. In particular, the assumed reaction time value may be inserted as a numerical value. Additionally or alternatively, different mathematical correlation functions may be used for different assumed reaction time values.

Thus, for a first time range of the assumed reaction time value, a first mathematical correlation function may be used for determining the analyte concentration from the color formation value, and for a second time range of the assumed reaction time value, a second mathematical correlation function may be used. As an example, a mathematical function "*f*" may be used for an assumed reaction time value *"between 13 and 45 seconds",* wherein a mathematical function "g" may be used for an assumed reaction time value *"between 45 and 120 seconds".* As an example, in case the assumed reaction time value indicates *"longer than 120 seconds"* the analytical measurement may even be aborted, e.g. by displaying an error message on a display of the mobile device. Further, in particular, the function "g" may be determinable from the function "*f*" corrected by using a predetermined correction function, e.g. applying a correction value. Other forms of functions may be used for representing the correlation between the analyte concentration and the color formation value may be used, for example look-up tables or interpolations. As an example, the assumed reaction time value may be used for selecting the function, such as "f" or "g", to be used for determining the analyte concentration from the color formation value, wherein, however, in the calculation of the analyte concentration itself, i.e. when using and/or applying the mathematical function, i.e. "f" or "g", the assumed reaction time value may not be used or may even be irrelevant.

The term "assessing the plausibility" as used herein is a broad term and is to be given its ordinary and customary meaning to a person of ordinary skill in the art and is not to be limited to a special or customized meaning. The term specifically may refer, without limitation, to a process of quantitatively and/or qualitatively determining a credibility and/or probability of an element and/or data. As an example, the plausibility may be assessed by using one or more characteristic parameters and/or properties of the element and/or data. These one or more characteristic parameters and/or properties may, individually or according to a predetermined combination, be compared with one or more conditions. Thus, as an example, the plausibility of the assumed reaction time value may be assessed by using one or more characteristic parameters and/or properties of an expected characteristic, specifically by using the color expectation range as will be outlined in further detail below. Specifically, for the assumed reaction time value, the respective color formation value may be compared to the color expectation range, e.g. to one or more comparative values, reference values or standard values, wherein the comparison may be a qualitative or quantitative comparison and may result in a binary result such as "plausible" or "not plausible"/"implausible". Additionally or alternatively, however, the comparison may result in a quantitative result, such as a figure indicating a degree of plausibility.

The term "color expectation range" as used herein is a broad term and is to be given its ordinary and customary meaning to a person of ordinary skill in the art and is not to be limited to a special or customized meaning. The term specifically may refer, without limitation, to a continuous and/or discrete scope of a color space within which a color is expected and/or predicted to be for a range of predefined reaction time values, i.e. for a predefined reaction time value range. Thus, as an example, the color expectation range may be one or more of a one-dimensional, two-dimensional or three-dimensional area of a color space comprising at least one of comparative values, reference values and/or standard values, for a predefined reaction time value range. Thus, the color expectation range may be or may comprise a continuous area and/or corridor comprising at least one expected color value. Additionally or alternatively, however, the color expectation range may be or may comprise a conglomerate of discrete expected colors, e.g. comprising discrete comparative values, reference values and/or standard values. **In** particular, the color expectation range, specifically a two-dimensional or three-dimensional color expectation range, may have an arbitrary form and/or shape.

Specifically, for deriving the color expectation range, in-time capture time values and tolerably delayed capture time values may be used in order to set and/or predefine the reaction time value range on which later, i.e. in a measurement method, the plausibility assessment may be based.

The term "determination method of determining at least one color expectation range for assessing the plausibility of an assumed reaction time value used in an analytical measurement based on a color formation reaction", also simply referred to as a "determination method", as used herein is a broad term and is to be given its ordinary and customary meaning to a person of ordinary skill in the art and is not to be limited to a special or customized meaning. The term specifically may refer, without limitation, to a method of determining the color expectation range as defined above, specifically according to a predefined reaction time value, i.e. to in-time capture time values and tolerably delayed capture time values. Specifically, the term may refer to a method by which, i.e. as a result, at least one range defining an expected range of color formation values is determined and/or ascertained, in particular for in-time capture time values and tolerably delayed capture time values as will be outlined further below.

The term "capture time value" as used herein is a broad term and is to be given its ordinary and customary meaning to a person of ordinary skill in the art and is not to be limited to a special or customized meaning. The term specifically may refer, without limitation, to a numerical indication of a time that has passed between a sample application, such as the sample application in step b) of the determination method, and a capturing of an image, such as the capturing of images in step c). Specifically, the capture time value may be specific to one captured image. **In** particular, the capture time value may refer to the actual time that has passed between sample application and image capturing and may thus differ from the assumed reaction time value used in the analytical measurement.

The term "in-time capture time value" as used herein is a broad term and is to be given its ordinary and customary meaning to a person of ordinary skill in the art and is not to be limited to a special or customized meaning. The term specifically may refer, without limitation, to a capture time value that is within a predefined time range considered to be "punctual" and "in time". **In** particular, all capture time values that fulfill a predefined punctuality requirement, i.e. are within the predefined time range, may be referred to as "in-time capture time values". **In** particular, the in-time capture time values may be or may comprise capture time values within a predefined time range, such as within a previously set and/or defined time range. As an example, the predefined time range and/or predefined punctuality requirement may be dependent on a characteristic of the color change detection reaction performed by the optical test strip, i.e. by the chemical in the reagent test region of the optical test strip. Thus, as an example, the predefined time range may be between 5 seconds and 300 seconds. Specifically, the predefined time range may be between 5 seconds and 180 seconds. More specifically, the predefined time range may be between 5 seconds and 120 seconds. More specifically, the predefined time range may be between 10 seconds and 60 seconds. More specifically, the predefined time range may be between 13 seconds and 45 seconds. Other predefined time ranges for defining capture time values to be in-time capture time values may be possible.

The term "delayed capture time value" as used herein is a broad term and is to be given its ordinary and customary meaning to a person of ordinary skill in the art and is not to be limited to a special or customized meaning. The term specifically may refer, without limitation, to a capture time value comprising a predefined time lag, e.g. a predefined delay. **In** particular, the delayed capture time value may refer to a capture time value that is delayed with regard to the predefined time range considered to be "punctual" and/or "in time". Specifically, all capture time values that are greater than an upper limit of the predefined range, e.g. outside of the predefined range in a direction of increasing time, may be referred to as "delayed capture time values". **In** particular, the delayed capture time value may refer to a numerical indication of a sum of a delay, specifically a predefined and/or known delay, and the upper limit of the predefined range and/or predefined punctuality requirement for the in-time capture time value. Thus, the delayed capture time value may differ from the in-time capture time value by at least the delay. As an example, throughout the specification, the delay may be referred to as *d,* wherein *d* may be or may comprise a positive or negative value.

The term "training set" as used herein is a broad term and is to be given its ordinary and customary meaning to a person of ordinary skill in the art and is not to be limited to a special or customized meaning. The term specifically may refer, without limitation, to a plurality of elements having known and/or predetermined differences and/or similarities. In particular, the training set may be used for training a trainable model, such as a model that can be further trained and/or updated based on additional information, e.g. gathered from the training set.

The term "training set of optical test strips" may specifically refer, without limitation, to a plurality of optical test strips as defined above. In particular, the training set of optical test strips as provided in step a) comprises a plurality of optical test strips, e.g. of optical test strips identical in construction and/or design.

The term "training set of samples of bodily fluids" as used herein is a broad term and is to be given its ordinary and customary meaning to a person of ordinary skill in the art and is not to be limited to a special or customized meaning. The term specifically may refer, without limitation, to a plurality of samples having at least one known and/or predetermined analyte concentration, e.g. determined in a laboratory environment. In particular, the training set of samples of bodily fluids may comprise a plurality of samples of bodily fluids as defined above, wherein for one or more of the plurality of samples the quantitative and/or qualitative analytical measurement result value, such as the concentration of at least one analyte within the sample, is known. Specifically, the number of samples comprised in the training set of samples may differ from the number of optical test strips in the training set of optical test strips. Alternatively, however, the number of samples in the training set of samples may equal the number of optical test strips in the training set of optical test strips.

The term "mobile device" as used herein is a broad term and is to be given its ordinary and customary meaning to a person of ordinary skill in the art and is not to be limited to a special or customized meaning. The term specifically may refer, without limitation, to a mobile electronics device, more specifically to a mobile communication device such as a cell phone or smartphone. Additionally or alternatively, the mobile device may also refer to a tablet computer or another type of portable computer having at least one camera. Further, as will be outlined below, the mobile device may optionally comprise further elements, such as for example one or more processors.

The term "camera" as used herein is a broad term and is to be given its ordinary and customary meaning to a person of ordinary skill in the art and is not to be limited to a special or customized meaning. The term specifically may refer, without limitation, to a device having at least one imaging element configured for recording or capturing spatially resolved one-dimensional, two-dimensional or even three-dimensional optical data or information. As an example, the camera may comprise at least one camera chip, such as at least one CCD chip and/or at least one CMOS chip configured for recording images. As used herein, without limitation, the term "image" specifically may relate to data recorded by using a camera, such as a plurality of electronic readings from the imaging device, such as the pixels of the camera chip.

Consequently, the term "training set of images" may specifically relate to a plurality of images, i.e. to a plurality of image data recorded by using a camera, e.g. the camera as defined above. In particular, the training set of images may refer to a plurality of images, e.g. a stack of digital images, of at least one part of one or more of the reagent test regions of the training set of optical test strips. In particular, the training set of images comprises a first training subset of images and a second training subset of images. As an example, the training set of images may comprise images of each of the reagent test regions of the training set of optical test strips, wherein, for example, one or more of the samples of bodily fluid may have been applied to the reagent test regions, e.g. before image capturing. Specifically, the training set of images may be used for determining a training set of color formation values of the reagent test regions of the training set of optical test strips, such as a plurality of color formation values for the training set of optical test strips. One or more of the images of the training set of images may be of more than one reagent test region. Thus, as an example, the number of images of the training set of images may differ from the number of optical test strips in the training set of optical test strips. However, equal numbers of images in the training set of images and of optical test strips in the training set of optical test strips may also be possible. Specifically, as an example, the training set of images may for example comprise separate images for each of the reagent test regions of the training set of optical test strips.

In particular, each image of the training set of images may be captured at the in-time capture time values and/or the delayed capture time values. Specifically, the images of the training set of images comprised by the first training subset of images are captured at the in-time capture time values, wherein the in-time capture time values may be different for one or more images within the first training subset of images. Specifically, the in-time capture time values for one or more images of the first training subset of images may be known or even pre-set. Further, the images of the training set of images comprised by the second training subset of images are captured at the delayed capture time values, wherein the delayed capture time values may be different for one or more images within the second training subset of images. Specifically, the delayed capture time values for one or more images of the second training subset of images may be known or even pre-set. Thus, in particular, each image of the training set of images may be assigned and/or linked to at least one in-time capture time value or delayed capture time value.

The camera, besides the at least one camera chip or imaging chip, may comprise further elements, such as one or more optical elements, e.g. one or more lenses. As an example, the camera may be a fix-focus camera, having at least one lens, which is fixedly adjusted with respect to the camera. Alternatively, however, the camera may also comprise one or more variable lenses, which may be adjusted, automatically or manually. The invention specifically shall be applicable to cameras as usually used in mobile applications such as notebook computers, tablets or, specifically, cell phones such as smart phones. Thus, specifically, the camera may be part of a mobile device which, besides the at least one camera, comprises one or more data processing devices such as one or more data processors. Other cameras, however, are feasible.

The camera specifically may be a color camera. Thus, such as for each pixel, color information may be provided or generated, such as color values for three colors R, G, B, for example also referred to as color channels. A larger number of color values, is also feasible, such as four color values for each pixel, for example R, G, G, B. Color cameras are generally known to the skilled person. Thus, as an example, the camera chip may consist of a plurality of three or more different color sensors each, such as color recording pixels like one pixel for red (R), one pixel for green (G) and one pixel for blue (B). For each of the pixels, such as for R, G, B, values may be recorded by the pixels, such as digital values in the range of 0 to 255, depending on the intensity of the respective color. Instead of using color triples such as R, G, B, as an example, quadruples may be used, such as R, G, G, B. The color sensitivities of the pixels may be generated by color filters or by appropriate intrinsic sensitivities of the sensor elements used in the camera pixels. These techniques are generally known to the skilled person.

The term "processor" as used herein is a broad term and is to be given its ordinary and customary meaning to a person of ordinary skill in the art and is not to be limited to a special or customized meaning. The term specifically may refer, without limitation, to an arbitrary logic circuitry configured for performing basic operations of a computer or system, and/or, generally, to a device which is configured for performing calculations or logic operations. In particular, the processor may be configured for processing basic instructions that drive the computer or system. As an example, the processor may comprise at least one arithmetic logic unit (ALU), at least one floating-point unit (FPU), such as a math coprocessor or a numeric coprocessor, a plurality of registers, specifically registers configured for supplying operands to the ALU and storing results of operations, and a memory, such as an L1 and L2 cache memory. In particular, the processor may be a multicore processor. Specifically, the processor may be or may comprise a central processing unit (CPU). Additionally or alternatively, the processor may be or may comprise a microprocessor, thus specifically the processor's elements may be contained in one single integrated circuitry (IC) chip. Additionally or alternatively, the processor may be or may comprise one or more application-specific integrated circuits (ASICs) and/or one or more field-programmable gate arrays (FPGAs) or the like.

The processor, specifically the processor which may be used in step d) of the determination method, may, for example, be a separate processor, such as a stand-alone processor or a processor integrated into a computer or computer network, separate from the mobile device. Alternatively, however, the processor may be integrated into the mobile device used in step c) for capturing the training set of images. Thus, specifically, the processor may be a processor of the mobile device.

Step e) of the determination method may comprise deriving at least two color expectation ranges. Specifically, the at least two color expectation ranges may be complementary, e.g. sharing at least one border. Thus, as an example, the determination method may be configured for determining two complementary color expectation ranges.

As an example, for a first of the complementary color expectation ranges the delay, specifically a first delay referred to as *d₁,* may in particular not exceed a predefined acceptance threshold. The acceptance threshold may specifically throughout the specification be referred to as *dₐ.* The acceptance threshold *dₐ* may further be lower than the expiry threshold *dₘₐₓ.* Thus, specifically *d₁ < dₐ < dₘₐₓ.* Further, for a second of the complementary color expectation ranges the delay, specifically a second delay referred to as *d₂,* may be equal to *dₐ* or may be between the acceptance threshold *dₐ* and the expiry threshold *dₘₐₓ.* Thus, specifically *dₐ ≤ d₂* ≤ *dₘₐₓ.*

Consequently, step e) of the determination method may comprise deriving at least two complementary color expectation ranges, wherein the first color expectation range may be or may comprise colors, specifically color formation values, to be expected for an assumed reaction time value range comprising assumed reaction time values differing from the actual and/or real reaction time values by less than the acceptance threshold *dₐ,* specifically by less than an acceptable delay. In particular, the first color expectation range may be or may comprise colors, specifically color formation values, derived and/or determined from images captured at in-time capture time values and delayed capture time values, wherein the delay of the delayed capture time values is smaller than the acceptable delay. Further, the second color expectation range, e.g. derived in step e), may be or may comprise colors, specifically color formation values, to be expected for an assumed reaction time value range comprising assumed reaction time values differing from the actual and/or real reaction time value by at least the acceptance threshold *dₐ,* specifically by at least the acceptable delay, but by no more than the expiry threshold *dₘₐₓ,* specifically by no more than an expiry delay. In particular, the second color expectation range may be or may comprise colors, specifically color formation values, derived and/or determined from images captured at delayed capture time values, wherein the delay of the delayed capture time values is greater or equal to the acceptable delay and below or equal to the expiry delay. In other words, the first color expectation range may comprise color formation values to be expected for assumed reaction times differing from the actual and/or real reaction times by less than an acceptable delay, wherein the second color expectation range may comprise color formation values to be expected for assumed reaction times differing from the actual and/or real reaction times by at least the acceptable delay but by no more than the expiry delay.

Step d) may further comprise labelling the color formation values of the training set of color formation values with information on the capture time values, i.e. with the in-time capture time values or the delayed capture time values. Specifically, the labelling may be taken into consideration in step e). In particular, the information on the capture time values may comprise information on whether the image was taken at the in-time capture time value or the delayed capture time value, wherein for the delayed capture time value further information, specifically whether the delay is below or above the at least one predefined expiry threshold and optionally below or above the acceptance threshold, may be comprised.

In step e) the color expectation range may comprise at least 80% of the color formation values for the color formation of the reagent test region of the optical test strips of the training set of optical test strips determined from images captured at one or more of the in-time capture time values and the tolerably delayed capture time values. Specifically, in step e) the color expectation range may comprise at least 85% of the color formation values for the color formation of the reagent test region of the optical test strips of the training set of optical test strips determined from images captured at one or more of the in-time capture time values and the tolerably delayed capture time values. More specifically, in step e) the color expectation range may comprise at least 90%, of the color formation values for the color formation of the reagent test region of the optical test strips of the training set of optical test strips determined from images captured at one or more of the in-time capture time values and the tolerably delayed capture time values. More specifically, in step e) the color expectation range may comprise at least 95%, of the color formation values for the color formation of the reagent test region of the optical test strips of the training set of optical test strips determined from images captured at one or more of the in-time capture time values and the tolerably delayed capture time values. More specifically, in step e) the color expectation range may comprise at least 97%, of the color formation values for the color formation of the reagent test region of the optical test strips of the training set of optical test strips determined from images captured at one or more of the in-time capture time values and the tolerably delayed capture time values. More specifically, in step e) the color expectation range may comprise at least 99%, of the color formation values for the color formation of the reagent test region of the optical test strips of the training set of optical test strips determined from images captured at one or more of the in-time capture time values and the tolerably delayed capture time values.

As an example, the color expectation range may be or may comprise at least one polygon. Specifically, the color expectation range may be or may comprise a two-dimensional polygon, the edges of which may correspond to the color formation values in a two-dimensional color space, such as in a color plane of at least two colors. Additionally or alternatively, the color expectation range may be or may comprise a three-dimensional polyhedron, the edges of which may correspond to the color formation values in a three-dimensional color space. Further additionally or alternatively, instead of the edges corresponding to the color formation values, the edges may be spaced apart from the color formation values, such that at least 80%, specifically at least 85%, more specifically at least 90%, more specifically at least 95%, more specifically at least 97%, more specifically at least 99%, of the color formation values for the color formation of the reagent test region of the optical test strips of the training set of optical test strips determined from images captured at one or more of the in-time capture time values and the tolerably delayed capture time values may be enclosed by the polygon and/or polyhedron.

In particular, the deriving in step e) may comprise determining an envelope comprising at least 80%, specifically at least 85%, more specifically at least 90%, more specifically at least 95%, more specifically at least 97%, more specifically at least 99%, of the color formation values for the color formation of the reagent test region of the optical test strips of the training set of optical test strips determined from images captured at one or more of the in-time capture time values and the tolerably delayed capture time values and further expanding the envelope by a predetermined safety factor.

The term "envelope" as used herein is a broad term and is to be given its ordinary and customary meaning to a person or ordinary skill in the art and is not to be limited to a special or customized meaning. The term specifically may refer, without limitation, to an element and/or entity enclosing and/or covering at least one set of data. In particular, the envelope may enclose, i.e. in at least one color plane and/or color space, at least 80%, specifically at least 85%, more specifically at least 90%, more specifically at least 95%, more specifically at least 97%, more specifically at least 99%, of the color formation values for the color formation of the reagent test region of the optical test strips of the training set of optical test strips determined from images captured at one or more of the in-time capture time values and the tolerably delayed capture time values, wherein the envelope may be mathematically and/or graphically determined.

In particular, step e) may comprise, specifically in a subsequent step, expanding the envelope by a predetermined safety factor. The safety factor may specifically be predetermined and/or pre-set, such as a safety factor taking into consideration the size and/or volume of the envelope. As an example, the safety factor may be or may comprise a function dependent on the envelope, i.e. on the size and/or volume of the envelope. As an example, the envelope may be expanded such that a size and/or volume of the expanded envelope may exceed the size and/or volume of the envelope by at least a factor of 1.1, specifically by at least 1.2, more specifically by at least 1.5. As an example, the envelope may be expanded such that 99% or even 100% of the color formation values determined from images captured at one or more of the in-time capture time values and the tolerably delayed capture time values are enclosed. Additionally or alternatively, the safety factor may take into consideration a deviation of the distribution of the color formation values, such as the standard deviation σ of the color formation values for the color formation of the reagent test region determined from images captured at one or more of the in-time capture time values and the tolerably delayed capture time values. Thus, as an example, the envelope may be expanded by a factor to cover a range of at least 4σ, preferably of at least 5σ, more preferably of at least 6σ.

The expansion may specifically be an equally distributed expansion, such as an even and/or uniform expansion of the envelope. As an example, a uniform expansion of the envelope may be performed in case the color formation values are equally distributed within the envelope. An unevenly distributed expansion, however, is also possible. Specifically, the envelope may be non-uniformly and/or unevenly expanded, i.e. based and/or depending on a weighted distribution of the color formation values within the envelope.

Step e) may specifically comprise using at least one machine-learning algorithm, specifically by training a trainable model by using the training set of color formation values. The term "machine-learning algorithm" as used herein is a broad term and is to be given its ordinary and customary meaning to a person of ordinary skill in the art and is not to be limited to a special or customized meaning. The term specifically may refer, without limitation, to a mathematical model being trainable by using records of training data, such as comprising training input data and corresponding training output data. **In** particular, the training output data of the record of training data may be the result that is expected to be produced by the machine-learning algorithm when being given the training input data of the same record of training data as input. As an example, the deviation between this expected result and the actual result produced by the algorithm may be observed and rated by means of a "loss function". This loss function may be used as a feedback for adjusting the parameters of the internal processing chain of the machine-learning algorithm. The machine-learning algorithm may comprise decision trees, naive bayes classifications, nearest neighbors, neural networks, convolutional neural networks, generative adversarial net-works, support vector machines, linear regression, logistic regression, random forest and/or gradient boosting algorithms. As an example, the machine-learning algorithm may be trained by using the training set of color formation values as input data and the corresponding information on the in-time capture time value and/or delayed capture time value as output data. Specifically, the output data may be or may comprise information on whether the respective color formation value for the color formation of the reagent test region of the optical test strips of the training set of optical test strips was determined from images captured at the in-time capture time values and the tolerably delayed capture time values or at an expired capture time, i.e. later than tolerable for safely determining an analyte concentration. Specifically, the machine-learning algorithm may be or may comprise a trainable color expectation range model, i.e. describing a shape and/or form of the color expectation range, wherein the feedback for adjusting the parameters of the internal processing chain of the model may specifically be based on a quantitative or qualitative determination whether the color formation values corresponding to the in-time capture time values and/or tolerably delayed capture time values, i.e. the color formation values for acceptable capture time values, are within the color expectation range. Other forms of feedback may be possible.

In step d) the color formation values may specifically be determined for at least two color channels, such as for at least two color channels selected from the group consisting of: a green color channel (G), a blue color channel (B) and a red color channel (R). In particular, in step e) the color expectation range may be derived for the at least two color channels for which the color formation values are determined in step d).

Further, the method may comprise step f) of attaching at least one optical test strip of the training set of optical test strips to a color reference card comprising a plurality of color reference fields having known reference color values. In particular, step f) may be performed before step c). Furthermore, at least one image of the set of images captured in step c) may further show at least part of the color reference card, specifically one or more of the color reference fields. The term "color reference card" as used herein is a broad term and is to be given its ordinary and customary meaning to a person of ordinary skill in the art and is not to be limited to a special or customized meaning. The term specifically may refer, without limitation, to an arbitrary item having, disposed therein or disposed thereon, such as on at least one surface, the plurality of color reference fields having known color properties or optical properties, such as having a plurality of colored fields having known reference color values. Further, the color reference card may comprise a plurality of gray reference fields having known gray levels. As an example, the color reference card may be a flat card comprising at least one substrate having, on at least one surface and/or disposed therein, the plurality of color reference fields having known color values and the plurality of gray reference fields having known gray levels. The substrate, specifically, may have a flat surface with the color reference fields and the gray reference fields disposed thereon. The substrate, as an example, may be or may comprise one or more of a paper substrate, a cardboard substrate, a plastic substrate, a ceramic substrate or a metal substrate. Laminate substrates are also possible. The substrate, as an example, may be sheet-like or flexible. It shall be noted, however, that the substrate may also be implemented into an article of use, such as into a wall of a box, a vial, a container, a medical consumable, such as a test strip, or the like. The color reference card may also fully or partially be integrated into the optical test strip. The at least one image of at least the part of the reagent test region of the optical test strip may fully or partially comprise an image of at least one part of the color reference card.

In a further aspect of the invention, a measurement method of performing an analytical measurement based on a color formation reaction by using a mobile device having a camera and a processor is disclosed. The measurement method comprises the following steps that, as an example, may be performed in the given order. It shall be noted, however, that a different order may generally also be possible. Further, it may also be possible to perform one or more of the method steps once or repeatedly. Further, it may also be possible to perform two or more of the method steps simultaneously or in a timely overlapping fashion. The measurement method may comprise further method steps that are not listed.

The measurement method comprises:
i) providing at least one optical test strip having at least one reagent test region;
ii) applying a sample of bodily fluid to the reagent test region of the optical test strip;
iii) capturing, by using the camera, at least one image of at least a part of the reagent test region having the bodily fluid applied thereto;
iv) determining an assumed reaction time value corresponding to the time that has passed between an assumed time of application of the sample in step ii) and the capturing of the image in step iii);
v) determining, specifically by using the processor, a color formation value of at least one color channel for a color formation of the reagent test region by using the image;
vi) comparing, for the at least one color channel, the color formation value to at least one color expectation range determined by performing the determination method according to any one of the preceding claims;
vii) if the color formation value is outside of the at least one color expectation range, considering the assumed reaction time value to be not plausible and aborting the measurement method; and
viii) if the color formation value is inside of the color expectation range, considering the assumed reaction time value to be plausible and determining a concentration of analyte in the sample of bodily fluid by using the color formation value and, optionally, the assumed reaction time value.

For the definitions of the measurement method, reference may be made to the description of the determination method described above or as will be outlined in further detail below. Specifically, for performing the measurement method, the same types of optical test strips may be used as in the determination method outlined above. Thus, as an example, the optical test strip provided in step i) of the measurement method may be of the same or at least similar type as the plurality of optical test strips of the training set of optical test strips provided in step a) of the determination method as described above or as will be described in more detail below. The sample of bodily fluid, however, may be a sample of bodily fluid from a user, the analyte concentration of which is to be determined and thus, may previously be unknown.

**In** particular, in step vii), the assumed reaction time value may differ from an actual capture time value by more than an acceptable difference. Consequently, in step viii) the assumed reaction time value may differ from the actual capture time value by no more than an acceptable difference.

Step vi) may comprise comparing the color formation value to at least one first color expectation range and to at least one second color expectation range determined by performing the determination method as described herein. Specifically, in step vii) if the color formation value is outside of both the first color expectation range and the second color expectation range, the assumed capture time may be considered to be not plausible and the measurement method may be aborted. Further, in step viii) different algorithms may be used for determining the concentration of the analyte from the color formation value for color formation values in the at least one first color expectation range and in the at least one second color expectation range.

**In** particular, the measurement method may in step viii) comprise:
- if the color formation value is inside the first color expectation range, the assumed reaction time value is considered to be plausible and the concentration of the analyte is determined by using the color formation value and the assumed reaction time value; and
- if the color formation value is inside the second color expectation range, the assumed reaction time value is considered to be plausible and the concentration of the analyte is determined by using the color formation value and a pre-defined delay for the second color expectation range added to the assumed reaction time value.

The measurement method may further comprise an intensity check, such as a step of checking whether an intensity of the color formation value is above or below at least one intensity threshold. **In** particular, in case the color formation value is outside of a predefined intensity range, e.g. below a lower intensity threshold or above an upper intensity threshold, the measurement method may be aborted.

The measurement method may further comprise step ix) of capturing, by using the camera, at least one image of at least a part of the regent test region without having the bodily fluid applied thereto. **In** particular, step ix) may be performed before step ii). Thus, as an example, the measurement method may comprise the capturing of at least one second image, specifically a blank image of the reagent test region without having the sample of bodily fluid applied thereto.

Further, the measurement method may comprise step x) of attaching the optical test strip to a color reference card comprising a plurality of color reference fields having known reference color values. The color reference card may specifically be of the same type as the color reference card optionally used in the determination method as described above. **In** particular, step x) may be performed before step iii), and, optionally, before step ii) of the measurement method, wherein specifically, the image captured in step iii) may further show at least part of the color reference card, specifically one or more of the color reference fields.

The applying in step ii) may further comprise confirming, specifically by a user, that the sample of bodily fluid is or has been applied to the regent test region of the optical test strip. Thus, as an example, the application of step ii) may be or may comprise a confirmation of the user that the sample of bodily fluid has been applied, i.e. by pushing a button and/or other form of confirmation, e.g. by interacting with the mobile device. Specifically, step ii) of the measurement method may comprise prompting a user to perform one or more of applying the sample of bodily fluid to the reagent test region of the optical test strip and confirming application of the sample of bodily fluid to the reagent test regions of the optical test strip. **In** particular, when performing step ii), the user may be prompted to apply the sample of bodily fluid and/or the user may be prompted to confirm sample application, e.g. by providing corresponding instructions on a display of the mobile device and/or as audio instructions.

**In** a further aspect of the invention, a determination system for determining at least one color expectation range for assessing the plausibility of an assumed reaction time value used in an analytical measurement based on a color formation reaction, comprising:
A) at least one mobile device having at least one camera;
B) a training set of optical test strips, each optical test strip having a reagent test region;
C) a training set of samples of bodily fluids comprising a plurality of samples of bodily fluids; and
D) at least one processor, the processor being configured for:
   - retrieving a training set of images, the training set of images comprising images, captured with the camera, of at least one part of at least some of the reagent test regions of the training set of optical test strips having the sample of bodily fluid applied thereto captured at in-time capture time values, the capture time value referring to the time that has passed between the application of the sample and the capturing of the image, specifically the first training subset of images captured in step c) of the determination method above, the training set of images further comprising images, captured with the camera, of at least one part of at least some of the reagent test regions of the training set of optical test strips having the sample of bodily fluid applied thereto captured at delayed capture time values, specifically the second training subset of images captured in step c) of the determination method above;
   - determining a training set of color formation values from the images of the training set of images, comprising color formation values of at least one color channel for the color formation of the reagent test region of the optical test strips of the training set of optical test strips for in-time capture time values and for delayed capture time values; and
   - deriving the at least one color expectation range for the at least one color channel from the training set of color formation values, the color expectation range defining an expected range of color formation values for in-time capture time values and tolerably delayed capture time values, wherein for the tolerably delayed capture time values the delay *d* does not exceed at least one predefined expiry threshold *dₘₐₓ.*

In particular, the delay *d* may be smaller or equal to the expiry threshold *dₘₐₓ.* Thus, as an example, d ≤ *dₘₐₓ.* Alternatively, the delay *d* may be smaller to the expiry threshold *dₘₐₓ.* Thus, as an alternative option, *d < dₘₐₓ.*

The processor of the determination system may, for example, be a separate processor, such as a stand-alone processor or a processor integrated into a computer or computer network, separate from the at least one mobile device. Alternatively, however, the processor may be integrated into the mobile device.

The determination system may further comprise:
E) at least one color reference card configured for having the optical test strip releasably attached thereto, the color reference card comprising a plurality of color reference fields having known reference color values, wherein the images of the training set of images further show at least part of the color reference card, specifically one or more of the color reference fields.

For most of the definitions of the determination system, reference may be made to the description of the determination method as described above or as will be outlined in further detail below. In particular, the determination system may be configured for performing the determination method as described herein. In particular, the determination system may be configured for performing at least steps d) and e) of the determination method as described herein.

Further disclosed and proposed herein is a computer program comprising instructions which, when the program is executed by a determination system, specifically by the determination system as described herein, cause the determination system to carry out at least steps d) and e) of the determination method as also described herein. Thus, specifically the computer program may comprise computer-executable instructions for performing the determination method according to the present invention in one or more of the embodiments enclosed herein when the instructions are executed on a determination system, i.e. on the at least one processor of the determination system, for example integrated into a computer or computer network. Specifically, the computer program may be stored on a computer-readable data carrier and/or on a computer-readable storage medium.

Thus, further disclosed and proposed herein is a computer-readable storage medium comprising instructions which, when executed by a determination system, specifically by the determination system as described herein, cause the determination system to carry out at least steps d) and e) of the determination method as also described herein.

As used herein, the terms "computer-readable data carrier" and "computer-readable storage medium" specifically may refer to non-transitory data storage means, such as a hardware storage medium having stored thereon computer-executable instructions. The computer-readable data carrier or storage medium specifically may be or may comprise a storage medium such as a random-access memory (RAM) and/or a read-only memory (ROM).

Further disclosed and proposed herein is a computer program product having program code means, in order to perform the determination method according to the present invention in one or more of the embodiments enclosed herein when the program is executed on a determination system, i.e. on the at least one processor of the determination system, for example integrated into a computer or computer network. Specifically, the program code means may be stored on a computer-readable data carrier and/or on a computer-readable storage medium.

Further disclosed and proposed herein is a data carrier having a data structure stored thereon, which, after loading into a computer or computer network, such as into a working memory or main memory of the computer or computer network, may execute the determination method according to one or more of the embodiments disclosed herein.

Further disclosed and proposed herein is a computer program product with program code means stored on a machine-readable carrier, in order to perform the determination method according to one or more of the embodiments disclosed herein, when the program is executed on a computer or computer network. As used herein, a computer program product refers to the program as a tradable product. The product may generally exist in an arbitrary format, such as in a paper format, or on a computer-readable data carrier and/or on a computer-readable storage medium. Specifically, the computer program product may be distributed over a data network.

Furthermore, disclosed and proposed herein is a modulated data signal which contains instructions readable by a computer system or computer network, for performing the determination method according to one or more of the embodiments disclosed herein.

**In** a further aspect of the invention, a mobile device having at least one camera and at least one processor is disclosed. The mobile device is configured for performing at least steps iv) to viii) of the measurement method as described herein. Thus, for definitions of terms reference is made to the description above, specifically with regard to the measurement method, as described herein.

Further disclosed and proposed herein is a computer program comprising instructions which, when the program is executed by a mobile device having a camera and a processor, specifically by the mobile device as described herein, cause the mobile device to carry out at least steps iv) to viii) of the measurement method. Thus, specifically, the computer program may comprise computer-executable instructions for performing the measurement method according to the present invention in one or more of the embodiments enclosed herein when the instructions are executed on the processor of the mobile device. Specifically, the computer program may be stored on a computer-readable data carrier and/or on a computer-readable storage medium.

Thus, further disclosed and proposed herein is a computer-readable storage medium comprising instructions which, when executed by a mobile device having a camera and a processor, specifically by the mobile device as described herein, cause the mobile device to carry out at least steps iv) to viii) of the measurement method as also described herein.

Further disclosed and proposed herein is a computer program product having program code means, in order to perform the measurement method according to the present invention in one or more of the embodiments enclosed herein when the program is executed on a mobile device, i.e. on the at least one processor of the mobile device, for example integrated into a computer or computer network. Specifically, the program code means may be stored on a computer-readable data carrier and/or on a computer-readable storage medium.

Further disclosed and proposed herein is a data carrier having a data structure stored thereon, which, after loading into a computer or computer network, such as into a working memory or main memory of the computer or computer network, may execute the measurement method according to one or more of the embodiments disclosed herein.

Further disclosed and proposed herein is a computer program product with program code means stored on a machine-readable carrier, in order to perform the measurement method according to one or more of the embodiments disclosed herein, when the program is executed on a computer or computer network. As used herein, a computer program product refers to the program as a tradable product. The product may generally exist in an arbitrary format, such as in a paper format, or on a computer-readable data carrier and/or on a computer-readable storage medium. Specifically, the computer program product may be distributed over a data network.

Furthermore, disclosed and proposed herein is a modulated data signal which contains instructions readable by a computer system or computer network, for performing the measurement method according to one or more of the embodiments disclosed herein.

**In** a further aspect of the invention, a kit for determining the concentration of at least one analyte in a sample of a bodily fluid, specifically a sample of bodily fluid of a user, is disclosed. The kit comprises the mobile device as described above, specifically the mobile device being configured for performing at least steps iv) to viii) of the measurement method as described herein. The kit further comprises at least one optical test strip having at least one reagent test region.

The methods and devices according to the present invention provide a large number of advantages over similar methods and devices known in the art. Specifically, compared to methods and devices known in the art, the methods and devices as described herein may increase measurement safety. Specifically, measurement safety may be increased by providing an effective fail safe mechanism, for example, due to the color formation value having to be within the color expectation range, in particular when performing the measurement method, in order for the concentration of the analyte to be determined. Thus, specifically, the provided methods and devices may increase measurement safety, since not all and any measured color value is converted into an analyte concentration, e.g. into a blood glucose value. Instead, the present methods and devices may allow for a detection of incorrect user information on a time of application of the sample.

Furthermore, measurement safety and accuracy may be improved by the proposed methods and devices compared to known methods and devices. The methods may specifically comprise preventing the determining of the concentration of the analyte in the bodily fluid if the plausibility assessment may not be fulfilled. Therefore, false and/or biased results of the analytical measurement may become more unlikely.

Furthermore, the proposed methods and devices may allow for an increased user handling and/or improved user friendliness of analytical measurement, i.e. by allowing a safe analytical measurement to be performed by only capturing one image, instead of capturing at least two images. Specifically, the overall time necessary for performing the analytical measurement may be decreased compared to known methods and devices.

### Short description of the Figures

Further optional features and embodiments will be disclosed in more detail in the subsequent description of embodiments, preferably in conjunction with the dependent claims. Therein, the respective optional features may be realized in an isolated fashion as well as in any arbitrary feasible combination, as the skilled person will realize. The scope of the invention is not restricted by the preferred embodiments. The embodiments are schematically depicted in the Figures. Therein, identical reference numbers in these Figures refer to identical or functionally comparable elements.

In the Figures:
- Figure 1: shows an embodiment of a determination system and an embodiment of a kit;
- Figure 2: shows a graphical illustration of an embodiment of a determination method;
- Figures 3 and 4: show embodiments of color expectation ranges;
- Figures 5 and 6: show graphical illustrations of different embodiments of a measurement method;
- Figure 7: shows a graphical illustration of a diagram indicating a relationship between actual blood glucose values and determined blood glucose values by using both common methods and systems and using present methods and systems for the determination of blood glucose concentrations.

### Detailed description of the embodiments

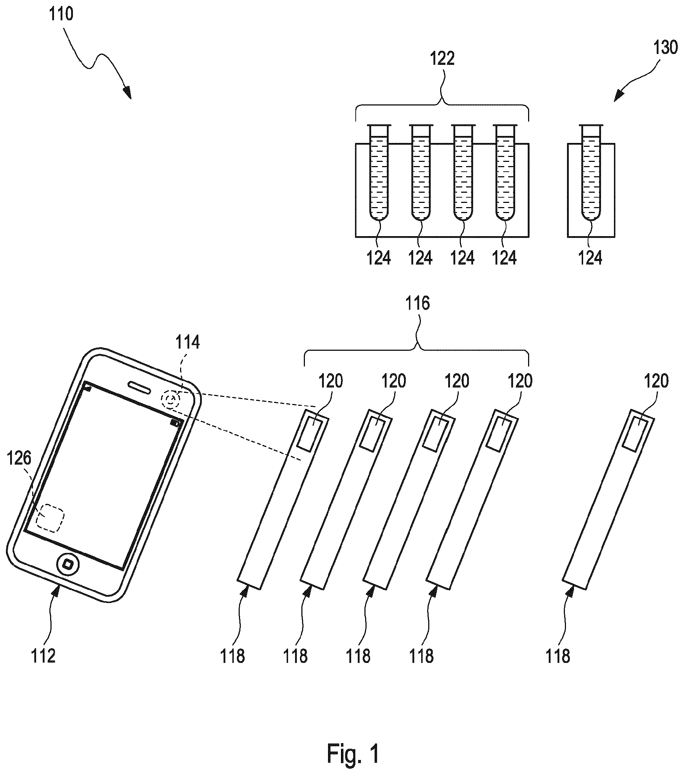

In Figure 1, an embodiment of a determination system 110 is illustrated. The determination system 110 comprises at least one mobile device 112 having at least one camera 114. Further, the determination system 110 comprises a training set of optical test strips 116. The training set of optical test strips 116 comprises a plurality of optical test trips 118, each optical test strip 118 having a reagent test region 120. Further, the determination system 110 comprises a training set of samples of body fluids 122 comprising a plurality of samples of bodily fluids 124. In particular, for each of the samples of bodily fluids 124 and the training set of samples of body fluid 122 and analyte concentration, e.g. a glucose concentration, may be known. Furthermore, the determination system 110 comprises at least one processor 126. The processor 126 of the determination system 110 may, for example, be a separate processor 126. Alternatively however, and as illustrated in Figure 1, the processor 126 may be integrated into the mobile device 112, such that the processor 126 may be a processor 126 of the mobile device 112.

In the determination system 110, the processor 126 is configured for retrieving a training set of images comprising images captured with the camera 114 of the mobile device 112. In particular, the training set of images comprises a first training subset of images and a second training subset of images, wherein the images of the first training subset of images are captured at an in-time capture time value and wherein the images of the second training subset of images are captured at a delayed capture time value. Thus, the processor 126 is configured for retrieving both the first training subset of images and the second training subset of images, specifically from the camera 114. Further, in the determination system 110, the processor 126 is configured for determining a training set of color formation values from the images of the training set of images. The training set of color formation values comprises color formation values of at least one color channel for the color formation of the reagent test region 120 each of the optical test strips 118 of the training set of optical test strips 116 for in-time capture time values and for delayed capture time values. Furthermore, in the determination system 110, the processor 126 is configured for deriving at least one color expectation range 128 for the at least one color channel from the training set of color formation values, wherein the color expectation range 128 defines an expected range of color formation values for in-time capture time values and tolerably delayed capture time values, wherein for the tolerably delayed capture time values the delay *d* does not exceed at least one predefined expiry threshold *dₘₐₓ.*

Figure 1 also illustrates an embodiment of a kit 130 configured for determining the concentration of at least one analyte in a sample of bodily fluid 124. The sample of bodily fluid 124 may specifically comprise a bodily fluid of a user, such as a sample having an unknown analyte concentration. In Figure 1, such a sample is exemplary illustrated on the far right of the figure. The kit 130 comprises at least one optical test strip 118 having at least one reagent test region 120. In Figure 1, such an optical test strip 118, i.e. a singular optical test strip 118, is exemplary illustrated on the far right of the figure. Further, the kit 130 comprises a mobile device 112 having at least one camera 114 and at least one processor 126. As an example, the mobile device 112 of the kit 130 may be the same mobile device 112 of the determination system 110. Alternatively however, the kit 130 and the determination system 110 each comprise their own mobile device 114, i.e. distinct and separate mobile devices 112.

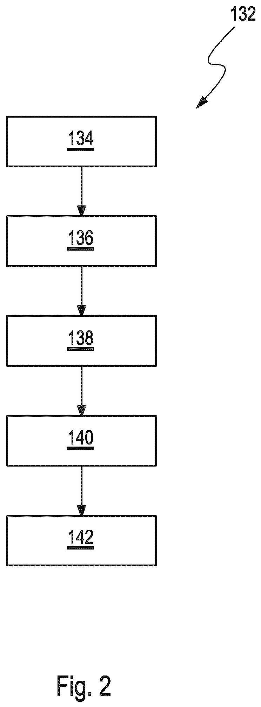

The determination system 110 and may specifically be configured for at least partially performing a determination method 132. An exemplary embodiment of the determination method 132 is shown in Figure 2. The determination method 132 is configured for determining at least one color expectation range 128 for assessing the plausibility of an assumed reaction time value used in an analytical measurement based on a color formation reaction. The determination method 132 comprises the following steps:
a) (denoted with reference number 134) providing a training set of optical test strips 116, each optical test strip 118 having a reagent test region 120;
b) (denoted with reference number 136) providing a training set of samples of bodily fluids 122 and applying at least one of the samples of bodily fluid 124 to the reagent test region 120 of each optical test strip 118 of the training set of optical test strips 116;
c) (denoted with reference number 138) capturing, by at least one mobile device 112 having at least one camera 114, a training set of images comprising:
   - a first training subset of images, the first training subset of images comprising images of at least one part of at least some of the reagent test regions 120 of the training set of optical test strips 116 having the sample of bodily fluid 124 applied thereto captured at in-time capture time values, the capture time value referring to the time that has passed between the application of the sample 124 in step b) and the capturing of the image;
   - at least one second training subset of images, the at least one second training subset of images comprising images of at least one part of at least some of the reagent test regions 120 of the training set of optical test strips 116 having the sample of bodily fluid 124 applied thereto captured at delayed capture time values;
d) (denoted with reference number 140) determining, specifically by using at least one processor 126, more specifically a processor 126 of the mobile device 112, a training set of color formation values from the images of the training set of images, the training set of color formation values comprising color formation values of at least one color channel for the color formation of the reagent test region 120 of the optical test strips 118 of the training set of optical test strips 116 for in-time capture time values and for delayed capture time values; and
e) (denoted with reference number 142) deriving the at least one color expectation range 128 for the at least one color channel from the training set of color formation values, the color expectation range 128 defining an expected range of color formation values for in-time capture time values and tolerably delayed capture time values, wherein for the tolerably delayed capture time values the delay d does not exceed at least one predefined expiry threshold *dₘₐₓ.*

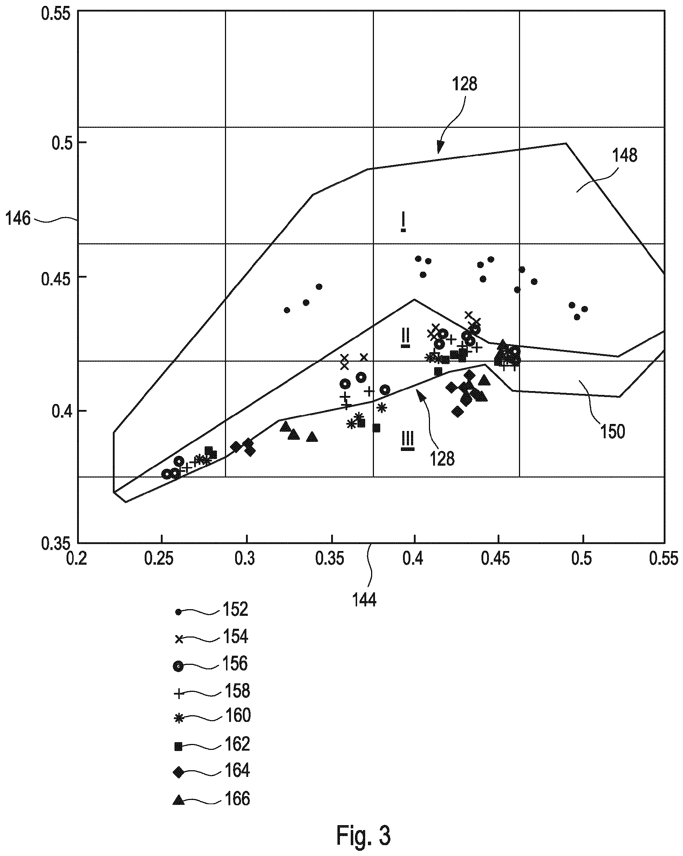

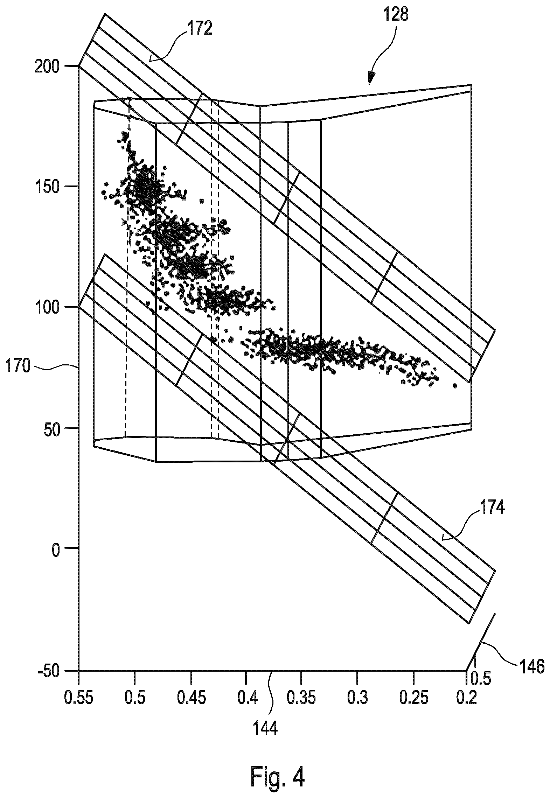

In Figures 3 and 4, exemplary embodiments of color expectation ranges 128 are shown. As illustrated in Figure 3, the color expectation range 128 may, for example, be or may comprise a polygon, such as a two-dimensional polygon in a color plane. For example, on a horizontal axis the color plane may show color values in a red color channel 144, wherein on a vertical axis the color plane may show color values in a green color channel 146. Further, in Figure 3, color formation values of an exemplary training set of color formation values are illustrated. As an example, the color expectation range 128 may comprise at least 80% of the color formation values for the color formation of the reagent test region of the optical test strips of the training set of optical test strips determined from images captured at one or more of the in-time capture time values and the tolerably delayed capture time values, i.e. at least 80% of these color formation values may be enclosed by the polygon shape of the color expectation range 128.

Specifically, and as is exemplary illustrated in Figure 3, at least two complementary color expectation ranges 128, i.e. a first color expectation range 148 and a second color expectation range 150, may be determined. The first color expectation range 148 may be or may comprise colors, specifically color formation values, to be expected for an assumed reaction time value differing from the actual and/or real reaction time value by no more than an acceptable delay, wherein the acceptable delay may correspond to a predefined and/or predetermined acceptance threshold *dₐ.* The second color expectation range 150 may be or may comprise colors, specifically color formation values, to be expected for an assumed reaction time value differing from the actual and/or real reaction time value by more than the acceptable delay but by no more than an expiry delay, wherein the expiry delay may correspond to the predefined and/or predetermined expiry threshold *dₘₐₓ.* In particular, in the Figure 3, the first color expectation range 148 may also be denoted by "I" and the second color expectation range 150 may also be denoted by "II", wherein an area outside of both the first color expectation range 148 and the second color expectation range 150 may be denoted by "III".

Further, in Figure 3, color formation values are illustrated for the in-time capture time values and the delayed capture time values. In particular, color formation values corresponding to the in-time capture time value, such as *d=0 min,* are denoted with reference number 152. Similarly, color formation values corresponding to a delayed capture time value of *d* = *1 min* are denoted with reference number 154. Further, 156 corresponds to *d* = *2 min,* 158 corresponds to *d* = *3 min,* 160 corresponds to *d* = *4 min, 162* corresponds to *d* = *5 min,* 164 corresponds to *d* = *7.5 min* and 166 corresponds to *d* = *10 min.*

As an example, by the illustrated color expectation ranges 148 and 150, an acceptable delay may have been considered to be a delay of 2 min, i.e. *dₐ.* = *2min,* and an expiry delay may have been considered to be a delay of 6 min, i.e. *dₘₐₓ* = *6min.* Other selections of acceptable delays and/or expiry delays may be possible.

Additionally or alternatively, and as exemplarily illustrated in Figure 4, the color expectation range 128 may be or may comprise a three-dimensional form, such as a polyhedron. As an example, the color expectation range 128 may be formed by a polygon in a two-dimensional color space, such as a polygon in the color plane of the red color channel 144 and the green color channel 146, that has been expanded into a third dimension, for example referring to a blue color channel 170 or, alternatively, to an intensity value, i.e. a value in an intensity dimension, for example an absolute intensity value of the values of the green color channel 146. In particular, the color expectation range 128 may be limited in the direction of the blue color channel 170 by an upper plane 172 as well as by a lower plane 174. Other forms and/or geometries of the color expectation range 128 may be possible.

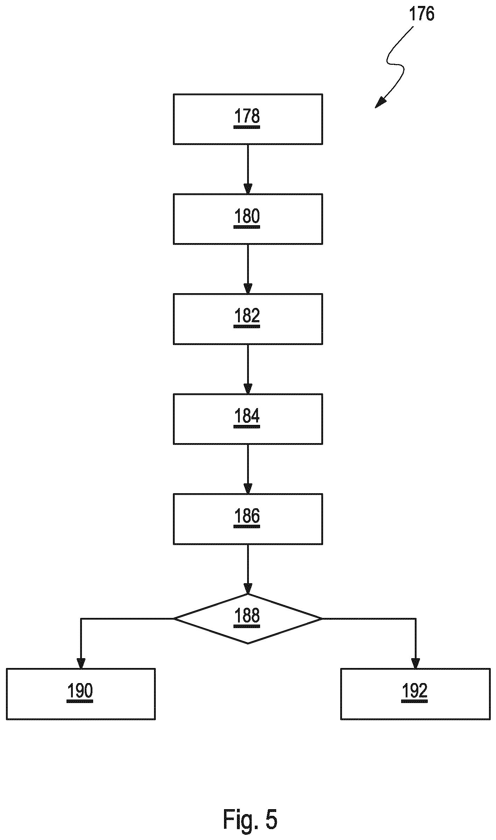

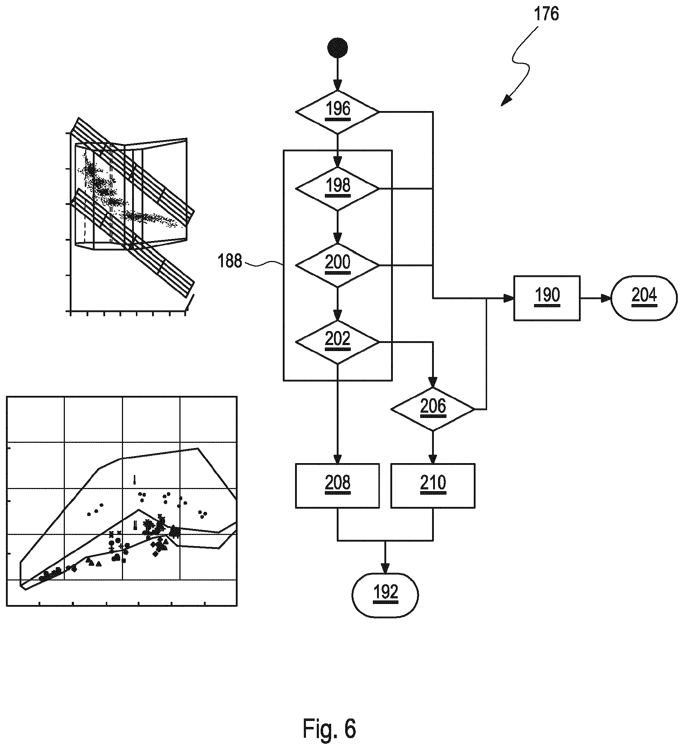

Specifically, the at least one color expectation range 128 may be used for assessing the plausibility of an assumed reaction time value used in an analytical measurement based on a color formation reaction. Such an analytical measurement may be performed by a measurement method 176. Exemplary embodiments of the measurement method 176 are illustrated in Figures 5 and 6. The measurement method 176 comprises the following steps:
i) (denoted with reference number 178) providing at least one optical test strip 118 having at least one reagent test region 120;
ii) (denoted with reference number 180) applying a sample of bodily fluid 124 to the reagent test region 120 of the optical test strip 118;
iii) (denoted with reference number 182) capturing, by using the camera 114, at least one image of at least a part of the reagent test region 120 having the bodily fluid 124 applied thereto;
iv) (denoted with reference number 184) determining an assumed reaction time value corresponding to the time that has passed between an assumed time of application of the sample in step ii) and the capturing of the image in step iii);
v) (denoted with reference number 186) determining, specifically by using the processor 126, a color formation value of at least one color channel for a color formation of the reagent test region 120 by using the image;
vi) (denoted with reference number 188) comparing, for the at least one color channel, the color formation value to at least one color expectation range determined by performing the determination method 132;
vii) (denoted with reference number 190) if the color formation value is outside of the at least one color expectation range 128, considering the assumed reaction time value to be not plausible and aborting the measurement method 176; and
viii) (denoted with reference number 192) if the color formation value is inside of the color expectation range 128, considering the assumed reaction time value to be plausible and determining a concentration of analyte in the sample of bodily fluid 124 by using the color formation value and, optionally, the assumed reaction time value.

Another embodiment of the measurement method 176 is illustrated in Figure 6. A starting point may be illustrated by a filled circle at the top of the figure. As an example, the measurement method 176 may further comprise an intensity check 196, such as a step of checking whether an intensity of the color formation value is above or below at least one intensity threshold. **In** particular, in case the color formation value is outside of a predefined intensity range, e.g. below a lower intensity threshold or above an upper intensity threshold, the measurement method may be aborted. Specifically, in case the color formation value fails the intensity check, the color formation value may be considered not plausible and thus, the measurement method 176 may be aborted.

It has to be noted that the color expectation range 128 may be a variable color expectation range, such as the basic points of the polygon shape and/or the parameters of the defining planes. For example, these basic points and/or parameters may be provided by means of metadata, which metadata are associated with a color card, to an application (on a user's mobile device) which allows to vary the basic points and/or parameters at a later point in time when needed. The variation of the color expectation range may be carried out in an optional method step before starting to carry out the measurement method 176.

As an example, in case the first color expectation range 148 corresponds to the first color expectation range 148 illustrated in Figure 4, step vi) 188 of the measurement method 176 may specifically comprise separate steps for comparing the color formation value to the first color expectation range 148. As an example, as denoted with reference number 198, it may be checked whether the color formation value is above the lower plane 174. Further, as denoted with reference number 200, it may be checked whether the color formation value is below the upper plane 172. Furthermore, as denoted with reference number 202, it may be checked whether the color formation value is within the first polygon shape "I" in the color plane defined by the red color channel 144 and the green color channel 146. **In** case the color formation value fails any of checks 198 to 202, the color formation value may be considered not plausible and the method may be aborted, according to step vii) 190. Further optionally, after aborting the method, the measurement method 176 may comprise showing an error message 204. The same checks may be performed for the second color expectation range 150. This step is illustrated in Figure 6 by reference number 206. For each of the first and the second color expectation ranges 148 and 150 different post processing code functions may be used for determining the concentration of the analyte. Thus, as denoted with reference numbers 208 and 210, a flag is set whether the color formation value is comprised in the first color expectation range 148, then the flag is set to "I" 208, or comprised in the second color expectation range 150, then the flag is set to "II" 210. As an example, for the first color expectation range 148, i.e. "I", a first mathematical correlation function, such as a mathematical function "*f*", may be used for determining the analyte concentration from the color formation value, and for the second color expectation range 150, i.e. "II", a second mathematical correlation function, a mathematical function "g", may be used.

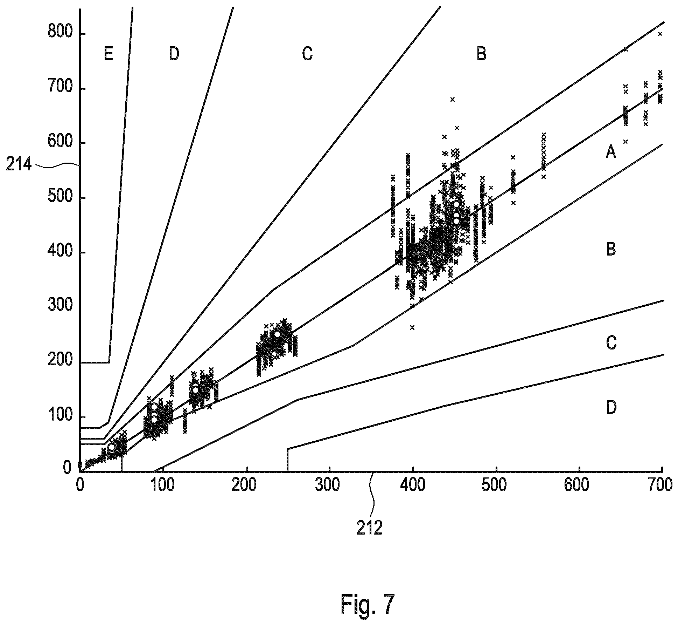

Figure 7 shows a graphical illustration of a diagram indicating a relationship between actual blood glucose values and determined blood glucose values by using both common methods and systems and using present methods and systems for the determination of blood glucose concentrations. In particular, the diagram illustrated in Figure 7 indicates the relationship between the actual blood glucose value in mg/dl 212 and the measured blood glucose value in mg/dl 214. In particular, values indicated by a cross show the relationship for normal measurements, i.e. measurements performed without provocation, specifically without wrongful and/or inappropriate handling procedures, wherein values indicated with circles show the relationship for provocational measurements, i.e. for measurements with wrongful and/or inappropriate user handling procedures, performed by using the present methods and devices. As can be seen, only valid results with a small error for the provocational measurements may be achieved.

Further, Figure 7 show regions A to E of an Error-Grid-Analysis, specifically regions A to E of the Parkes Error Grid, quantifying clinical accuracy of a determined blood glucose concentration compared to an actual blood glucose concentration. For example blood glucose values within:
- Region A contains values within 20% of the reference sensor;
- Region B contains values that are outside of 20% but would not lead to inappropriate treatment;
- Region C contains values leading to unnecessary treatment;
- Region D contains values indicating a potentially dangerous failure to detect hypoglycemia or hyperglycemia, and
- Region E contains values that would confuse treatment of hypoglycemia for hyperglycemia and vice versa.

For more information on the Error-Grid-Analysis reference may be made to Clarke WL, Cox D, Gonder-Frederick LA, Carter W, Pohl SL: Evaluating clinical accuracy of systems for self-monitoring of blood glucose. Diabetes Care 10:622-628,1987.

The measurements illustrated in Figure 7 are based on the same samples and assumed reaction times. In particular, the same samples and assumed reaction times have been used for determining the blood glucose values for both measurements.

Table 1 indicates the number of determined blood glucose values for a set of provocational measurements performed by using known methods and devices and the same measurements performed by using the present methods and devices. The set of provocational measurements, as a reference and/or for controlling purposes, further included a total number of 15 "normal" measurements performed without provocation, specifically without wrongful and/or inappropriate handling procedures, used as reference measurements.

**Table 1: Number of determined blood glucose values for both using known methods and devices and using present methods and devices**

| Legend | A | B | C | D | E |
|---|---|---|---|---|---|
| number of determined blood glucose values for known methods and devices | 89 | 14 | 2 | 2 | 0 |
| number of determined blood glucose values for present methods and devices | 19 | 0 | 0 | 0 | 0 |

Specifically, as can be seen in Table 1, the values of measurements performed by known methods and devices provide blood glucose values for almost all measurements, wherein in measurements according to the present invention, no blood glucose values are provided for assumed time spans considered to be not plausible. In particular, when using the present methods and devices, blood glucose values were determined for all of the 15 normal measurements used as a reference within the set of provocational measurements.

### List of reference numbers

- 110: determination system
- 112: mobile device
- 114: camera
- 116: training set of optical test strips
- 118: optical test strips
- 120: reagent test region
- 122: training set of samples of body fluids
- 124: samples of bodily fluids
- 126: processor
- 128: color expectation range
- 130: kit
- 132: determination method
- 134: step a)
- 136: step b)
- 138: step c)
- 140: step d)
- 142: step e)
- 144: red color channel
- 146: green color channel
- 148: first color expectation range
- 150: second color expectation range
- 152: color formation values corresponding to in-time capture time value *(d=0 min)*
- 154: *d=1 min*
- 156: *d=2 min*
- 158: *d=3 min*
- 160: *d=4 min*
- 162: *d=5 min*
- 164: *d=7.5 min*
- 166: *d=10 min*
- 170: blue color channel
- 172: upper plane
- 174: lower plane
- 176: measurement method
- 178: step i)
- 180: step ii)
- 182: step iii)
- 184: step iv)
- 186: step v)
- 188: step vi)
- 190: step vii)
- 192: step viii)
- 196: intensity check
- 198: check if color formation value is above lower plane
- 200: check if color formation value is below upper plane
- 202: check if color formation value is within polygon
- 204: error message
- 206: check if color formation value is within second color expectation range
- 208: set flag "I"
- 210: set flag "II"
- 212: actual blood glucose value in mg/dl
- 214: measured blood glucose value in mg/dl

## Claims

1. A determination method of determining at least one color expectation range (128) for assessing the plausibility of an assumed reaction time value used in an analytical measurement based on a color formation reaction, the method comprising:
a) providing a training set of optical test strips (116), each optical test strip (118) having a reagent test region (120);
b) providing a training set of samples of bodily fluids (122) and applying at least one of the samples of bodily fluid (124) to the reagent test region of each optical test strip (118) of the training set of optical test strips (116);
c) capturing, by at least one mobile device (112) having at least one camera (114), a training set of images comprising
- a first training subset of images, the first training subset of images comprising images of at least one part of at least some of the reagent test regions (120) of the training set of optical test strips (116) having the sample of bodily fluid applied thereto captured at in-time capture time values, the capture time value referring to the time that has passed between the application of the sample in step b) and the capturing of the image;
- at least one second training subset of images, the at least one second training subset of images comprising images of at least one part of at least some of the reagent test regions (120) of the training set of optical test strips (116) having the sample of bodily fluid applied thereto captured at delayed capture time values;
d) determining a training set of color formation values from the images of the training set of images, the training set of color formation values comprising color formation values of at least one color channel for the color formation of the reagent test region (120) of the optical test strips (118) of the training set of optical test strips (120) for in-time capture time values and for delayed capture time values; and
e) deriving the at least one color expectation range (128) for the at least one color channel from the training set of color formation values, the color expectation range defining an expected range of color formation values for in-time capture time values and tolerably delayed capture time values, wherein for the tolerably delayed capture time values the delay d does not exceed at least one predefined expiry threshold *dₘₐₓ.*

2. The determination method according to the preceding claim, wherein step e) comprises deriving at least two complementary color expectation ranges (148, 150).

3. The determination method according to the preceding claim, wherein for a first of the complementary color expectation ranges (148) a delay *d₁* does not exceed a predefined acceptance threshold *dₐ* lower than the expiry threshold *dₘₐₓ,* specifically *d₁* < *dₐ* < *dₘₐₓ,* and for a second of the complementary color expectation ranges (150) a delay *d₂* is equal to *dₐ* or between the acceptance threshold *dₐ* and the expiry threshold *dₘₐₓ,* specifically *dₐ ≤ d₂* ≤ *dₘₐₓ.*

4. The determination method according to any one of the preceding claims, wherein step d) further comprises labelling the color formation values of the training set of color formation values with information on the capture time values.

5. The determination method according to any one of the preceding claims, wherein in step e) the color expectation range (128) comprises at least 80%, specifically at least 85%, more specifically at least 90%, more specifically at least 95%, more specifically at least 97%, more specifically at least 99%, of the color formation values for the color formation of the reagent test region of the optical test strips of the training set of optical test strips determined from images captured at one or more of the in-time capture time values and the tolerably delayed capture time values.

6. The determination method according to any one of the preceding claims, wherein the deriving in step e) comprises determining an envelope comprising at least 80% of the color formation values for the color formation of the reagent test region (120) of the optical test strips (118) of the training set of optical test strips (116) determined from images captured at one or more of the in-time capture time values and the tolerably delayed capture time values and further expanding the envelope by a predetermined safety factor.

7. The determination method according to any one of the preceding claims, wherein step e) comprises using at least one machine-learning algorithm.

8. A measurement method of performing an analytical measurement based on a color formation reaction by using a mobile device (112) having a camera (114) and a processor (126), the method comprising:
i) providing at least one optical test strip (118) having at least one reagent test region (120);
ii) applying a sample of bodily fluid (124) to the reagent test region (120) of the optical test strip (118);
iii) capturing, by using the camera (114), at least one image of at least a part of the reagent test region (120) having the bodily fluid applied thereto;
iv) determining an assumed reaction time value corresponding to the time that has passed between an assumed time of application of the sample in step ii) and the capturing of the image in step iii);
v) determining a color formation value of at least one color channel for a color formation of the reagent test region (120) by using the image;
vi) comparing, for the at least one color channel, the color formation value to at least one color expectation range (128) determined by performing the determination method according to any one of the preceding claims;
vii) if the color formation value is outside of the at least one color expectation range (128), considering the assumed reaction time value to be not plausible and aborting the measurement method; and
viii) if the color formation value is inside of the color expectation range (128), considering the assumed reaction time value to be plausible and determining a concentration of analyte in the sample of bodily fluid by using the color formation value.

9. The measurement method according to the preceding claim, wherein step vi) comprises comparing the color formation value to at least one first color expectation range (148) and to at least one second color expectation range (150) determined by performing the determination method according to any one of claims 2 to 7, and wherein in step vii) if the color formation value is outside of both the first color expectation range and the second color expectation range, the assumed capture time is considered to be not plausible and the measurement method is aborted, wherein in step viii) different algorithms are used for determining the concentration of the analyte from the color formation value for color formation values in the at least one first color expectation range and in the at least one second color expectation range.

10. The measurement method according to the preceding claim, wherein in step viii):
- if the color formation value is inside the first color expectation range (148), the assumed reaction time value is considered to be plausible and the concentration of the analyte is determined by using the color formation value and the assumed reaction time value; and
- if the color formation value is inside the second color expectation range (150), the assumed reaction time value is considered to be plausible and the concentration of the analyte is determined by using the color formation value and a predefined delay for the second color expectation range added to the assumed reaction time value.

11. The measurement method according to any one of the two preceding claims, wherein the method further comprises step ix) of capturing, by using the camera (114), at least one image of at least a part of the reagent test region (120) without having the bodily fluid applied thereto, wherein step ix) is performed before step ii).

12. The measurement method according to any one of the two preceding claims, wherein the method further comprises step x) of attaching the optical test strip (118) to a color reference card comprising a plurality of color reference fields having known reference color values, wherein step x) is performed before step iii, and wherein the image captured in step iii) further shows at least part of the color reference card.

13. A determination system (110) for determining at least one color expectation range (128) for assessing the plausibility of an assumed reaction time value used in an analytical measurement based on a color formation reaction, comprising:
A) at least one mobile device (112) having at least one camera (114);
B) a training set of optical test strips (116), each optical test strip (118) having a reagent test region (120);
C) a training set of samples of bodily fluids (126) comprising a plurality of samples of bodily fluids (128); and
D) at least one processor (126), the processor (126) being configured for:
- retrieving a training set of images, the training set of images comprising images, captured with the camera (114), of at least one part of at least some of the reagent test regions (120) of the training set of optical test strips (116) having the sample of bodily fluid (124) applied thereto captured at in-time capture time value, the capture time value referring to the time that has passed between the application of the sample and the capturing of the image, the training set of images further comprising images, captured with the camera (114), of at least one part of at least some of the reagent test regions (120) of the training set of optical test strips (116) having the sample of bodily fluid (124) applied thereto captured at delayed capture time values;
- determining a training set of color formation values from the images of the training set of images, comprising color formation values of at least one color channel for the color formation of the reagent test region (120) of the optical test strips (118) of the training set of optical test strips (116) for in-time capture time values and for delayed capture time values; and
- deriving the at least one color expectation range (128) for the at least one color channel from the training set of color formation values, the color expectation range (128) defining an expected range of color formation values for in-time capture time values and tolerably delayed capture time values, wherein for the tolerably delayed capture time values the delay d does not exceed at least one predefined expiry threshold *dₘₐₓ.*

14. The determination system (110) according to the preceding claim, wherein the determination system further comprises:
E) at least one color reference card configured for having the optical test strip releasably attached thereto, the color reference card comprising a plurality of color reference fields having known reference color values, wherein the images of the training set of images further show at least part of the color reference card, specifically one or more of the color reference fields.

15. The determination system (110) according to any one of the two preceding claims, wherein the determination system is configured for performing at least steps d) and e) of the determination method according to any one of the preceding claims referring to a determination method.

16. A mobile device (112) having at least one camera (114) and at least one processor (126), the mobile device (112) being configured for performing at least steps iv) to viii) of the measurement method according to any one of the preceding claims referring to a measurement method.

17. A kit (130) for determining the concentration of at least one analyte in a sample of a bodily fluid (124), the kit (130) comprising the mobile device (112) according to the preceding claim, the kit (130) further comprising at least one optical test strip (118) having at least one reagent test region (120).

## Patentansprüche

1. Bestimmungsverfahren zur Bestimmung mindestens eines Farberwartungsbereiches (128) zur Bewertung der Plausibilität eines angenommenen Reaktionszeitwertes, der bei einer analytischen Messung auf der Basis einer Farbbildungsreaktion verwendet wird, wobei das Verfahren umfasst:
a) Bereitstellen eines Trainingssatzes von optischen Teststreifen (116), wobei jeder optische Teststreifen (118) einen Reagenztestbereich (120) aufweist;
b) Bereitstellen eines Trainingssatzes von Körperflüssigkeitsproben (122) und Aufbringen mindestens einer der Körperflüssigkeitsproben (124) auf den Reagenztestbereich jedes optischen Teststreifens (118) des Trainingssatzes von optischen Teststreifen (116);
c) Erfassen eines Trainingssatzes von Bildern durch mindestens ein mobiles Gerät (112) mit mindestens einer Kamera (114), umfassend
- einen ersten Trainingsteilsatz von Bildern, wobei der erste Trainingsteilsatz von Bildern Bilder von mindestens einem Teil von mindestens einigen der Reagenztestbereiche (120) des Trainingssatzes von optischen Teststreifen (116) umfasst, auf welche die Körperflüssigkeitsprobe aufgebracht wurde, die zu rechtzeitigen Erfassungszeitwerten erfasst wurde, wobei sich der Erfassungszeitwert auf die Zeit bezieht, die zwischen dem Aufbringen der Probe in Schritt b) und dem Erfassen des Bildes vergangen ist;
- mindestens einen zweiten Trainingsteilsatz von Bildern, wobei der mindestens eine zweite Trainingsteilsatz von Bildern Bilder von mindestens einem Teil von mindestens einigen der Reagenztestbereiche (120) des Trainingssatzes von optischen Teststreifen (116) umfasst, auf welche die Körperflüssigkeitsprobe aufgebracht wurde, die zu verzögerten Erfassungszeitwerten erfasst wurde;
d) Bestimmen eines Trainingssatzes von Farbbildungswerten aus den Bildern des Trainingssatzes von Bildern, wobei der Trainingssatz von Farbbildungswerten Farbbildungswerte von mindestens einem Farbkanal für die Farbbildung des Reagenztest (120) der optischen Teststreifen (118) des Trainingssatzes von optischen Teststreifen (120) für rechtzeitige Erfassungszeitwerte und für verzögerte Erfassungszeitwerte umfasst; und
e) Ableiten des mindestens einen Farberwartungsbereiches (128) für den mindestens einen Farbkanal aus dem Trainingssatz von Farbbildungswerten, wobei der Farberwartungsbereich einen erwarteten Bereich von Farbbildungswerten für rechtzeitige Erfassungszeitwerte und tolerierbar verzögerte Erfassungszeitwerte definiert, wobei für die tolerierbar verzögerten Erfassungszeitwerte die Verzögerung *d* mindestens einen vordefinierten Ablaufschwellenwert *dₘₐₓ* nicht überschreitet.

2. Bestimmungsverfahren nach dem vorhergehenden Anspruch, wobei Schritt e) das Ableiten von mindestens zwei komplementären Farberwartungsbereichen (148, 150) umfasst.

3. Bestimmungsverfahren nach dem vorhergehenden Anspruch, wobei für einen ersten der komplementären Farberwartungsbereiche (148) eine Verzögerung *d₁* einen vordefinierten Akzeptanzschwellenwert *dₐ* nicht überschreitet, der niedriger als der Ablaufschwellenwert *dₘₐₓ* ist, insbesondere *d₁*<*dₐ*<*dₘₐₓ,* und für einen zweiten der komplementären Farberwartungsbereiche (150) eine Verzögerung *d₂* gleich *dₐ* oder zwischen dem Akzeptanzschwellenwert *dₐ* und dem Ablaufschwellenwert *dₘₐₓ* ist, insbesondere *dₐ*≤*d₂*≤*dₘₐₓ.*

4. Bestimmungsverfahren nach einem der vorhergehenden Ansprüche, wobei Schritt d) ferner das Kennzeichnen der Farbbildungswerte des Trainingssatzes von Farbbildungswerten mit Informationen über die Erfassungszeitwerte umfasst.

5. Bestimmungsverfahren nach einem der vorhergehenden Ansprüche, wobei in Schritt e) der Farberwartungsbereich (128) mindestens 80%, insbesondere mindestens 85%, insbesondere mindestens 90%, insbesondere mindestens 95%, insbesondere mindestens 97%, insbesondere mindestens 99% der Farbbildungswerte für die Farbbildung des Reagenztestbereiches der optischen Teststreifen des Trainingssatzes von optischen Teststreifen umfasst, die aus Bildern bestimmt wurden, die zu einem oder mehreren der rechtzeitigen Erfassungszeitwerte und der tolerierbar verzögerten Erfassungszeitwerte erfasst wurden.

6. Bestimmungsverfahren nach einem der vorhergehenden Ansprüche, wobei das Ableiten in Schritt e) das Bestimmen einer Hüllkurve umfasst, die mindestens 80 % der Farbbildungswerte für die Farbbildung des Reagenztestbereiches (120) der optischen Teststreifen (118) des Trainingssatzes von optischen Teststreifen (116) umfasst, die aus Bildern bestimmt wurden, die zu einem oder mehreren der rechtzeitigen Erfassungszeitwerte und der tolerierbar verzögerten Erfassungszeitwerte erfasst wurden, und ferner das Erweitern der Hüllkurve um einen vorbestimmten Sicherheitsfaktor umfasst.

7. Bestimmungsverfahren nach einem der vorhergehenden Ansprüche, wobei Schritt e) die Verwendung mindestens eines Algorithmus für maschinelles Lernen umfasst.

8. Messverfahren zur Durchführung einer analytischen Messung auf der Basis einer Farbbildungsreaktion unter Verwendung eines mobilen Geräts (112) mit einer Kamera (114) und einem Prozessor (126), wobei das Verfahren Folgendes umfasst:
i) Bereitstellen mindestens eines optischen Teststreifens (118) mit mindestens einem Reagenztestbereich (120);
ii) Aufbringen einer Körperflüssigkeitsprobe (124) auf den Reagenztestbereich (120) des optischen Teststreifens (118);
iii) Erfassen mindestens eines Bildes von mindestens einem Teil des Reagenztestbereiches (120), auf den die Körperflüssigkeit aufgebracht wurde, unter Verwendung der Kamera (114);
iv) Bestimmen eines angenommenen Reaktionszeitwertes, welcher der Zeit entspricht, die zwischen einer angenommenen Zeit des Aufbringens der Probe in Schritt ii) und dem Erfassen des Bildes in Schritt iii) vergangen ist;
v) Bestimmen eines Farbbildungswertes von mindestens einem Farbkanal für eine Farbbildung des Reagenztestbereiches (120) unter Verwendung des Bildes;
vi) Vergleichen des Farbbildungswertes mit mindestens einem Farberwartungsbereich (128), der durch Durchführen des Bestimmungsverfahrens nach einem der vorhergehenden Ansprüche bestimmt wird, für den mindestens einen Farbkanal;
vii) wenn der Farbbildungswert außerhalb des mindestens einen Farberwartungsbereiches (128) liegt, Betrachten des angenommenen Reaktionszeitwertes als nicht plausibel und Abbrechen des Messverfahrens; und
viii) wenn der Farbbildungswert innerhalb des Farberwartungsbereiches (128) liegt, Betrachten des angenommenen Reaktionszeitwertes als plausibel und Bestimmen einer Konzentration des Analyten in der Körperflüssigkeitsprobe unter Verwendung des Farbbildungswertes.

9. Messverfahren nach dem vorhergehenden Anspruch, wobei Schritt vi) das Vergleichen des Farbbildungswertes mit mindestens einem ersten Farberwartungsbereich (148) und mit mindestens einem zweiten Farberwartungsbereich (150) umfasst, der durch Durchführen des Bestimmungsverfahrens nach einem der Ansprüche 2 bis 7 bestimmt wird, und wobei in Schritt vii), wenn der Farbbildungswert außerhalb sowohl des ersten Farberwartungsbereiches als auch des zweiten Farberwartungsbereiches liegt, die angenommene Erfassungszeit als nicht plausibel betrachtet wird und das Messverfahren abgebrochen wird, wobei in Schritt viii) verschiedene Algorithmen zur Bestimmung der Konzentration des Analyten aus dem Farbbildungswert für Farbbildungswerte in dem mindestens einen ersten Farberwartungsbereich und in dem mindestens einen zweiten Farberwartungsbereich verwendet werden.

10. Messverfahren nach dem vorhergehenden Anspruch, wobei in Schritt viii):
- wenn der Farbbildungswert innerhalb des ersten Farberwartungsbereiches (148) liegt, der angenommene Reaktionszeitwert als plausibel betrachtet wird und die Konzentration des Analyten unter Verwendung des Farbbildungswertes und des angenommenen Reaktionszeitwertes bestimmt wird; und
- wenn der Farbbildungswert innerhalb des zweiten Farberwartungsbereiches (150) liegt, der angenommene Reaktionszeitwert als plausibel betrachtet wird und die Konzentration des Analyten unter Verwendung des Farbbildungswertes und einer vordefinierten Verzögerung für den zweiten Farberwartungsbereich bestimmt wird, die zu dem angenommenen Reaktionszeitwert addiert wird.

11. Messverfahren nach einem der beiden vorhergehenden Ansprüche, wobei das Verfahren ferner Schritt ix) des Erfassens, unter Verwendung der Kamera (114), mindestens eines Bildes von mindestens einem Teil des Reagenztestbereiches (120) umfasst, ohne dass die Körperflüssigkeit darauf aufgebracht wurde, wobei Schritt ix) vor Schritt ii) durchgeführt wird.

12. Messverfahren nach einem der beiden vorhergehenden Ansprüche, wobei das Verfahren ferner Schritt x) des Anbringens des optischen Teststreifens (118) an einer Farbreferenzkarte umfasst, die eine Vielzahl von Farbreferenzfeldern mit bekannten Referenzfarbwerten umfasst, wobei Schritt x) vor Schritt iii) durchgeführt wird und wobei das in Schritt iii) erfasste Bild ferner mindestens einen Teil der Farbreferenzkarte zeigt.

13. Bestimmungssystem (110) zur Bestimmung mindestens eines Farberwartungsbereiches (128) zur Bewertung der Plausibilität eines angenommenen Reaktionszeitwertes, der bei einer analytischen Messung auf der Basis einer Farbbildungsreaktion verwendet wird, umfassend:
A) mindestens ein mobiles Gerät (112) mit mindestens einer Kamera (114);
B) einen Trainingssatz von optischen Teststreifen (116), wobei jeder optische Teststreifen (118) einen Reagenztestbereich (120) aufweist;
C) einen Trainingssatz von Körperflüssigkeitsproben (126), der eine Vielzahl von Körperflüssigkeitsproben (128) umfasst; und
D) mindestens einen Prozessor (126), wobei der Prozessor (126) zu Folgendem konfiguriert ist:
- Abrufen eines Trainingssatzes von Bildern, wobei der Trainingssatz von Bildern mit der Kamera (114) erfasste Bilder von mindestens einem Teil von mindestens einigen der Reagenztestbereiche (120) des Trainingssatzes von optischen Teststreifen (116) umfasst, auf welche die Körperflüssigkeitsprobe (124) aufgebracht wurde, die zu einem rechtzeitigen Erfassungszeitwert erfasst wurde, wobei sich der Erfassungszeitwert auf die Zeit bezieht, die zwischen dem Aufbringen der Probe und dem Erfassen des Bildes vergangen ist, wobei der Trainingssatz von Bildern ferner mit der Kamera (114) erfasste Bilder von mindestens einem Teil von mindestens einigen der Reagenztestbereiche (120) des Trainingssatzes von optischen Teststreifen (116) umfasst, auf welche die Körperflüssigkeitsprobe (124) aufgebracht wurde, die zu verzögerten Erfassungszeitwerten erfasst wurde;
- Bestimmen eines Trainingssatzes von Farbbildungswerten aus den Bildern des Trainingssatzes von Bildern, umfassend Farbbildungswerte von mindestens einem Farbkanal für die Farbbildung des Reagenztestbereiches (120) der optischen Teststreifen (118) des Trainingssatzes von optischen Teststreifen (116) für rechtzeitige Erfassungszeitwerte und für verzögerte Erfassungszeitwerte; und
- Ableiten des mindestens einen Farberwartungsbereiches (128) für den mindestens einen Farbkanal aus dem Trainingssatz von Farbbildungswerten, wobei der Farberwartungsbereich (128) einen erwarteten Bereich von Farbbildungswerten für rechtzeitige Erfassungszeitwerte und tolerierbar verzögerte Erfassungszeitwerte definiert, wobei für die tolerierbar verzögerten Erfassungszeitwerte die Verzögerung *d* mindestens einen vordefinierten Ablaufschwellenwert *dₘₐₓ* nicht überschreitet.

14. Bestimmungssystem (110) nach dem vorhergehenden Anspruch, wobei das Bestimmungssystem ferner Folgendes umfasst:
E) mindestens eine Farbreferenzkarte, die so konfiguriert ist, dass der optische Teststreifen lösbar daran angebracht ist, wobei die Farbreferenzkarte eine Vielzahl von Farbreferenzfeldern mit bekannten Referenzfarbwerten umfasst, wobei die Bilder des Trainingssatzes von Bildern ferner mindestens einen Teil der Farbreferenzkarte zeigen, insbesondere eines oder mehrere der Farbreferenzfelder.

15. Bestimmungssystem (110) nach einem der beiden vorhergehenden Ansprüche, wobei das Bestimmungssystem zum Durchführen mindestens der Schritte d) und e) des Bestimmungsverfahrens nach einem der vorhergehenden Ansprüche, die sich auf ein Bestimmungsverfahren beziehen, konfiguriert ist.

16. Mobiles Gerät (112) mit mindestens einer Kamera (114) und mindestens einem Prozessor (126), wobei das mobile Gerät (112) zum Durchführen mindestens der Schritte iv) bis viii) des Messverfahrens nach einem der vorhergehenden Ansprüche, die sich auf ein Messverfahren beziehen, konfiguriert ist.

17. Kit (130) zum Bestimmen der Konzentration von mindestens einem Analyten in einer Körperflüssigkeitsprobe (124), wobei der Kit (130) das mobile Gerät (112) nach dem vorhergehenden Anspruch umfasst, wobei der Kit (130) ferner mindestens einen optischen Teststreifen (118) mit mindestens einem Reagenztestbereich (120) umfasst.

## Revendications

1. Méthode de détermination permettant de déterminer au moins un intervalle d'attente de couleur (128) pour évaluer la plausibilité d'une valeur supposée de temps de réaction utilisée dans une mesure analytique basée sur une réaction de formation de couleur, la méthode comprenant :
a) la fourniture d'un ensemble d'entraînement de bandelettes de test optiques (116), chaque bandelette de test optique (118) ayant une région de test réactive (120) ;
b) la fourniture d'un ensemble d'entraînement d'échantillons de fluides corporels (122) et l'application d'au moins l'un des échantillons de fluide corporel (124) sur la région de test réactive de chaque bandelette de test optique (118) de l'ensemble d'entraînement de bandelettes de test optiques (116) ;
c) la capture, par au moins un dispositif mobile (112) ayant au moins une caméra (114), d'un ensemble d'entraînement d'images comprenant
- un premier sous-ensemble d'entraînement d'images, le premier sous-ensemble d'entraînement d'images comprenant des images d'au moins une partie d'au moins certaines des régions de test réactives (120) de l'ensemble d'entraînement de bandelettes de test optiques (116) sur lesquelles est appliqué l'échantillon de fluide corporel capturées à des valeurs de temps de capture en temps réel, la valeur de temps de capture faisant référence au temps qui s'est écoulé entre l'application de l'échantillon à l'étape b) et la capture de l'image ;
- au moins un deuxième sous-ensemble d'entraînement d'images, l'au moins un deuxième sous-ensemble d'entraînement d'images comprenant des images d'au moins une partie d'au moins certaines des régions de test réactives (120) de l'ensemble d'entraînement de bandelettes de test optiques (116) sur lesquelles est appliqué l'échantillon de fluide corporel capturées à des valeurs de temps de capture retardées ;
d) la détermination d'un ensemble d'entraînement de valeurs de formation de couleur à partir des images de l'ensemble d'entraînement d'images, l'ensemble d'entraînement de valeurs de formation de couleur comprenant des valeurs de formation de couleur d'au moins un canal de couleur pour la formation de couleur de la région de test réactive (120) des bandelettes de test optiques (118) de l'ensemble d'entraînement de bandelettes de test optiques (120) pour les valeurs de temps de capture en temps réel et pour les valeurs de temps de capture retardées ; et
e) la dérivation de l'au moins un intervalle d'attente de couleur (128) pour l'au moins un canal de couleur à partir de l'ensemble d'entraînement de valeurs de formation de couleur, l'intervalle d'attente de couleur définissant un intervalle attendu de valeurs de formation de couleur pour les valeurs de temps de capture en temps réel et pour les valeurs de temps de capture retardées de manière tolérable, dans laquelle pour les valeurs de temps de capture retardées de manière tolérable le retard *d* ne dépasse pas au moins un seuil d'expiration prédéfini *dₘₐₓ.*

2. Méthode de détermination selon la revendication précédente, dans laquelle l'étape e) comprend la dérivation d'au moins deux intervalles d'attente de couleur complémentaires (148, 150).

3. Méthode de détermination selon la revendication précédente, dans laquelle pour un premier des intervalles d'attente de couleur complémentaires (148) un retard *d₁* ne dépasse pas un seuil d'acceptabilité *dₐ* prédéfini inférieur au seuil d'expiration *dₘₐₓ,* spécifiquement *d₁ < dₐ< dₘₐₓ,* et pour un deuxième des intervalles d'attente de couleur complémentaires (150) un retard *d₂* est égal à *dₐ* ou entre le seuil d'acceptabilité *dₐ* et le seuil d'expiration *dₘₐₓ,* spécifiquement *dₐ* ≤ *d₂* ≤ *dₘₐₓ.*

4. Méthode de détermination selon l'une quelconque des revendications précédentes, dans laquelle l'étape d) comprend en outre le marquage des valeurs de formation de couleur de l'ensemble d'entraînement de valeurs de formation de couleur avec les informations sur les valeurs de temps de capture.

5. Méthode de détermination selon l'une quelconque des revendications précédentes, dans laquelle à l'étape e) l'intervalle d'attente de couleur (128) comprend au moins 80 %, spécifiquement au moins 85 %, plus spécifiquement au moins 90 %, plus spécifiquement au moins 95 %, plus spécifiquement au moins 97 %, plus spécifiquement au moins 99 %, des valeurs de formation de couleur pour la formation de couleur de la région de test réactive des bandelettes de test optiques de l'ensemble d'entraînement de bandelettes de test optiques déterminées à partir des images capturées à une ou plusieurs parmi les valeurs de temps de capture en temps réel et les valeurs de temps de capture retardées de manière tolérable.

6. Méthode de détermination selon l'une quelconque des revendications précédentes, dans laquelle la dérivation à l'étape e) comprend la détermination d'une enveloppe comprenant au moins 80 % des valeurs de formation de couleur pour la formation de couleur de la région de test réactive (120) des bandelettes de test optiques (118) de l'ensemble d'entraînement de bandelettes de test optiques (116) déterminées à partir des images capturées à une ou plusieurs parmi les valeurs de temps de capture en temps réel et les valeurs de temps de capture retardées de manière tolérable et l'expansion supplémentaire de l'enveloppe par un facteur de sécurité prédéterminé.

7. Méthode de détermination selon l'une quelconque des revendications précédentes, dans laquelle l'étape e) comprend l'utilisation d'au moins un algorithme d'apprentissage machine.

8. Méthode de mesure permettant de réaliser une mesure analytique basée sur une réaction de formation de couleur en utilisant un dispositif mobile (112) ayant une caméra (114) et un processeur (126), la méthode comprenant :
i) la fourniture d'au moins une bandelette de test optique (118) ayant au moins une région de test réactive (120) ;
ii) l'application d'un échantillon de fluide corporel (124) sur la région de test réactive (120) de la bandelette de test optique (118) ;
iii) la capture, en utilisant la caméra (114), d'au moins une image d'au moins une partie de la région de test réactive (120) sur laquelle est appliqué le fluide corporel ;
iv) la détermination d'une valeur supposée de temps de réaction correspondant au temps qui s'est écoulé entre un temps supposé d'application de l'échantillon à l'étape ii) et la capture de l'image à l'étape iii) ;
v) la détermination d'une valeur de formation de couleur d'au moins un canal de couleur pour une formation de couleur de la région de test réactive (120) en utilisant l'image ;
vi) la comparaison, pour l'au moins un canal de couleur, de la valeur de formation de couleur avec au moins un intervalle d'attente de couleur (128) déterminé en réalisant la méthode de détermination selon l'une quelconque des revendications précédentes ;
vii) si la valeur de formation de couleur est à l'extérieur de l'au moins un intervalle d'attente de couleur (128), la considération que la valeur supposée de temps de réaction n'est pas plausible et l'interruption de la méthode de mesure ; et
viii) si la valeur de formation de couleur est à l'intérieur de l'intervalle d'attente de couleur (128), la considération que la valeur supposée de temps de réaction est plausible et la détermination d'une concentration de l'analyte dans l'échantillon de fluide corporel en utilisant la valeur de formation de couleur.

9. Méthode de mesure selon la revendication précédente, dans laquelle l'étape vi) comprend la comparaison de la valeur de formation de couleur avec au moins un premier intervalle d'attente de couleur (148) et avec au moins un deuxième intervalle d'attente de couleur (150) déterminés en réalisant la méthode de détermination selon l'une quelconque des revendications 2 à 7, et dans laquelle à l'étape vii) si la valeur de formation de couleur est à l'extérieur à la fois du premier intervalle d'attente de couleur et du deuxième intervalle d'attente de couleur, le temps de capture supposé est considéré comme n'étant pas plausible et la méthode de mesure est interrompue, dans laquelle à l'étape viii) différents algorithmes sont utilisés pour déterminer la concentration de l'analyte à partir de la valeur de formation de couleur pour les valeurs de formation de couleur dans l'au moins un premier intervalle d'attente de couleur et dans l'au moins un deuxième intervalle d'attente de couleur.

10. Méthode de mesure selon la revendication précédente, dans laquelle à l'étape viii) :
- si la valeur de formation de couleur est à l'intérieur du premier intervalle d'attente de couleur (148), la valeur supposée de temps de réaction est considérée comme étant plausible et la concentration de l'analyte est déterminée en utilisant la valeur de formation de couleur et la valeur supposée de temps de réaction ; et
- si la valeur de formation de couleur est à l'intérieur du deuxième intervalle d'attente de couleur (150), la valeur supposée de temps de réaction est considérée comme étant plausible et la concentration de l'analyte est déterminée en utilisant la valeur de formation de couleur et un retard prédéfini pour le deuxième intervalle d'attente de couleur ajouté à la valeur supposée de temps de réaction.

11. Méthode de mesure selon l'une quelconque des deux revendications précédentes, dans laquelle la méthode comprend en outre l'étape ix) de capture, en utilisant la caméra (114), d'au moins une image d'au moins une partie de la région de test réactive (120) sur laquelle n'est pas appliqué le fluide corporel, dans laquelle l'étape ix) est réalisée avant l'étape ii).

12. Méthode de mesure selon l'une quelconque des deux revendications précédentes, dans laquelle la méthode comprend en outre l'étape x) de fixation de la bandelette de test optique (118) à une carte de référence de couleur comprenant une pluralité de champs de référence de couleur ayant des valeurs de couleurs de référence connues, dans laquelle l'étape x) est réalisée avant l'étape iii, et dans laquelle l'image capturée à l'étape iii) révèle en outre au moins une partie de la carte de référence de couleur.

13. Système de détermination (110) permettant de déterminer au moins un intervalle d'attente de couleur (128) pour évaluer la plausibilité d'une valeur supposée de temps de réaction utilisée dans une mesure analytique basée sur une réaction de formation de couleur, comprenant :
A) au moins un dispositif mobile (112) ayant au moins une caméra (114) ;
B) un ensemble d'entraînement de bandelettes de test optiques (116), chaque bandelette de test optique (118) ayant une région de test réactive (120) ;
C) un ensemble d'entraînement d'échantillons de fluides corporels (126) comprenant une pluralité d'échantillons de fluides corporels (128) ; et
D) au moins un processeur (126), le processeur (126) étant configuré pour :
- récupérer un ensemble d'entraînement d'images, l'ensemble d'entraînement d'images comprenant des images, capturées avec la caméra (114), d'au moins une partie d'au moins certaines des régions de test réactives (120) de l'ensemble d'entraînement de bandelettes de test optiques (116) sur lesquelles est appliqué l'échantillon de fluide corporel (124) capturées à une valeur de temps de capture en temps réel, la valeur de temps de capture faisant référence au temps qui s'est écoulé entre l'application de l'échantillon et la capture de l'image, l'ensemble d'entraînement d'images comprenant en outre des images, capturées avec la caméra (114), d'au moins une partie d'au moins certaines des régions de test réactives (120) de l'ensemble d'entraînement de bandelettes de test optiques (116) sur lesquelles est appliqué l'échantillon de fluide corporel (124) capturées à des valeurs de temps de capture retardées ;
- déterminer un ensemble d'entraînement de valeurs de formation de couleur à partir des images de l'ensemble d'entraînement d'images, comprenant des valeurs de formation de couleur d'au moins un canal de couleur pour la formation de couleur de la région de test réactive (120) des bandelettes de test optiques (118) de l'ensemble d'entraînement de bandelettes de test optiques (116) pour les valeurs de temps de capture en temps réel et pour les valeurs de temps de capture retardées ; et
- dériver l'au moins un intervalle d'attente de couleur (128) pour l'au moins un canal de couleur à partir de l'ensemble d'entraînement de valeurs de formation de couleur, l'intervalle d'attente de couleur (128) définissant un intervalle attendu de valeurs de formation de couleur pour les valeurs de temps de capture en temps réel et les valeurs de temps de capture retardées de manière tolérable, dans lequel pour les valeurs de temps de capture retardées de manière tolérable le retard *d* ne dépasse pas au moins un seuil d'expiration prédéfini *dₘₐₓ.*

14. Système de détermination (110) selon la revendication précédente, dans lequel le système de détermination comprend en outre :
E) au moins une carte de référence de couleur configurée pour avoir la bandelette de test optique fixée de manière amovible à celle-ci, la carte de référence de couleur comprenant une pluralité de champs de référence de couleur ayant des valeurs de couleur de référence connues, dans lequel les images de l'ensemble d'entraînement d'images révèlent en outre au moins une partie de la carte de référence de couleur, spécifiquement un ou plusieurs des champs de référence de couleur.

15. Système de détermination (110) selon l'une quelconque des deux revendications précédentes, dans lequel le système de détermination est configuré pour réaliser au moins les étapes d) et e) de la méthode de détermination selon l'une quelconque des revendications précédentes faisant référence à une méthode de détermination.

16. Dispositif mobile (112) ayant au moins une caméra (114) et au moins un processeur (126), le dispositif mobile (112) étant configuré pour réaliser au moins les étapes iv) à viii) de la méthode de mesure selon l'une quelconque des revendications précédentes faisant référence à une méthode de mesure.

17. Kit (130) permettant de déterminer la concentration d'au moins un analyte dans un échantillon d'un fluide corporel (124), le kit (130) comprenant le dispositif mobile (112) selon la revendication précédente, le kit (130) comprenant en outre au moins une bandelette de test optique (118) ayant au moins une région de test réactive (120).
